# EUROPEAN PATENT APPLICATION

(11) **EP 2 000 543 A2**
(43) Date of publication of application: **10.12.2008**
(21) Application number: 07730326.1
(22) Date of filing: 15.02.2007
(51) Int. Cl.: C12Q 1/68, G01N 33/574, C07K 16/30

(54) **METHOD FOR THE MOLECULAR DIAGNOSIS OF PROSTATE CANCER AND KIT FOR IMPLEMENTING SAME**

(30) Priority: 15.02.2006 ES 200600348
(71) Applicant: Oryzon Genomics, S.A., 08028 Barcelona (ES); FUNDACIÓ PRIVADA CLÍNIC PER A LA RECERCA BIOMÈDICA, 08036 Barcelona (ES); Consejo Superior de Investigaciones Cientificas (CSIC), 28006 Madrid (ES)
(72) Inventor: THOMSON OKATSU, Timothy, E-08034 Barcelona (ES); BERMUDO GASCON, Raquel, E-08034 Barcelona (ES); RAMIREZ ORTIZ, Angel, E-08034 Barcelona (ES); ABIA, David, E-08034 Barcelona (ES); MATINEZ ALONSO, Carlos, E-08034 Barcelona (ES); FERNANDEZ RUIZ, Luis Pedro, E-08036 Barcelona (ES); FERRER FABREGA, Berta, E-08036 Barcelona (ES); CAMPO GÜERRI, Elias, E-08036 Barcelona (ES); ROSELL VIVES, Elisabet, E-08028 Barcelona (ES)
(74) Representative: Pons Arino, Angel
(86) International application number: PCT/ES2007/000085
(87) International publication number: WO 2007/093657

(57) **Abstract**

The invention relates to a method for the molecular diagnosis of prostate cancer, comprising the in vitro analysis of the overexpression or underexpresion of combinations of genes that can distinguish, with high statistical significance, tumorous prostate samples from non-tumorous prostate samples. The invention also relates to a kit for the molecular diagnosis of prostate cancer, which can perform the above-mentioned detection.

## Description

### FIELD OF THE INVENTION

The invention falls within the biotechnology sector and specifically within the field of methods for the diagnosis of prostate cancer. Accordingly, the present invention relates to a method for the molecular diagnosis of prostate cancer, comprising the *in vitro* analysis of the overexpression and underexpression of combinations of genes capable of differentiating between carcinomatous and noncarcinomatous prostate samples with high statistical significance. In particular, the present invention relates to a kit for the molecular diagnosis of prostate cancer capable of carrying out the aforementioned detection.

### BACKGROUND OF THE INVENTION

Prostate cancer (PC) is a neoplasia having one of the highest rates of mortality and morbidity in industrialized countries and has therefore considerable socioeconomic impact [1], for which reason it is the subject of intensive study. Despite this effort, and in contrast to other types of neoplasia, comparatively little of substance is known about the molecular factors determining its initiation, maintenance, and malignant progression. On the other hand, the most singular characteristic of PC - its high androgen dependence - can provide important keys to understanding some of the molecular mechanisms underlying the biology of this cancer.

As in other cancers, there exists a genetic susceptibility to PC, which is why so many studies have sought to discover the link between genetic loci and susceptibility to PC. These studies have yielded a multiplicity and diversity of genetic loci [2]. None of these loci and genes explains more than a small proportion of PC familial clusters and, which is more striking, none have been confirmed in independent replication studies. This could be explained by the great genetic heterogeneity of PC, such that several high-penetrance genes can be associated with different familial PC pedigrees, as well as by the high frequency of phenocopies, i.e. sporadic PCs that have found themselves included in familial PC studies, owing to their characteristics being indistinguishable. Alternately, it could be that no single gene is associated with susceptibility to PC, but that instead many genes are involved, each of them being of relatively low penetrance. An additional characteristic of familial PC is that it is not associated to any significant degree with other cancer types, with the possible exception of breast cancer and tumors of the CNS (central nervous system) in specific family clusters, which indicates that the gene or genes involved do not participate in generalized neoplastic syndromes but seem instead to be "organ-specific". However, PC has been used to study alterations in genes often associated with other neoplasias, such as TP53, BRCA1, PTEN, or repair genes affected, for example, in HNPCC (hereditary nonpolyposis colon cancer), and in fact few alterations or, as in the case of TP53 or PTEN, mutations that appear only at a late stage of tumor development have been found.

The fact that the PC susceptibility genes identified to date have been found altered in very few individuals and families stands in the way of an effective preventive approach to the problem. A related, though separate, question relates to early detection of PC. Determining the serum levels of PSA (prostate-specific antigen) in its various forms remains the most relevant reference for the detection and clinical follow-up of PC. Doubts arise when a differential diagnosis is required, or in cases where the PC is not accompanied by elevated PSA levels. This protein is a tissue marker and an androgen receptor signaling mechanism and not really a marker of malignity, so that, strictly speaking, its serum levels merely indicate the total mass of prostatic epithelial glands having the capacity to produce and secrete it. Elevated PSA levels are therefore observed not only in PC, but also in BPH (benign prostatic hyperplasia) and other benign prostatic processes, while, on the other hand, its production can sometimes be compromised in highly undifferentiated PCs, in which neoplastic prostate epithelial cells lose the capacity to express PSA.

This is why many laboratories are searching for new molecules that will offer greater specificity and sensitivity than PSA as a marker for the detection and follow-up of PC. The application of high-throughput (HT) techniques to the study of PC has allowed molecules to be identified that had previously not been associated with PC and which have shown themselves to be excellent malignity markers having a far superior differentiating capacity and specificity than PSA when detected in tissue [3]. Of these markers, the ones that stand out are alpha-methylacyl-CoA racemase (AMACR), hepsin (HPN), and fatty-acid synthase (FASN), which are expressed in large amounts in the majority of cases of PC, whereas, in contrast to PSA, its expression levels in normal prostate epithelium are minimal. Moreover, most malignant cells in PC lose their ability to express glutathione-S-transferase π (GSTP1) through hypermethylation of its promoter. Then again, as carcinomatous prostate glands have no basal cells, in PC there is decreased expression of genes and proteins characteristic of these cells, such as the high-molecular-weight keratins (e.g. CK5 or CK14) or the nuclear protein p63, a homolog of the cancer suppressor gene p53, which is expressed in the basal layers of several epithelia, including prostatic epithelium.

The availability of good reagents has allowed the use of some of these markers in clinically relevant applications such as determining levels in punch biopsy samples, thus demonstrating its usefulness in the diagnosis of doubtful cases of PC [4]. However, despite its great tumor specificity, none of the proteins mentioned is physiologically secreted by the prostate epithelium, which means that their determination in serum and other fluids - one of the greatest assets of PSA as an indicator of the mass of active prostate epithelium - does not give results that are fully consistent with their tissue determination. High-throughput studies are helping identify other secreted molecules that are expressed in anomalous quantities in PC. Determination of one or more of these proteins, even if they are not tissue-specific, in conjunction with the determination of PSA levels, is a promising avenue for developing tests of greater specificity and sensitivity.

There is therefore a need to identify subsets of markers for the diagnosis and prognosis of prostate cancer that are a significant improvement over existing ones. In this invention, new methods are provided for the molecular diagnosis of PC, having a high capacity to differentiate between carcinomatous and noncarcinomatous samples, based on the detection of the expression of a series of gene subsets described in the present invention, as well as kits capable of performing said methods and the uses of said kits for the diagnosis and prognosis of the disease. The use of expression levels of sets of two or more genes to differentiate between carcinomatous and noncarcinomatous samples makes it possible to achieve levels of statistical significance in such differentiation that is often not achievable with the determination of the expression level of a single gene.

### DESCRIPTION OF THE INVENTION

### Brief description of the invention

The present invention relates to a method for the molecular diagnosis of prostate cancer, comprising the *in vitro* analysis, in a test sample, of the expression level of at least one gene or subsets of at least two genes selected from the group of 60 genes comprising: TACSTD1, HPN, AMACR, APOC1, GJB1, PP3111, CAMKK2, ZNF85, SND1, NONO, ICA1, PYCR1, ZNF278, BIK, HOXC6, CDK5, LASS2, NME1, PRDX4, SYNGR2, SIM2, EIF3S2, NIT2, FOXA1, CX3CL1, SNAI2, GSTP1, DST, KRT5, CSTA, LAMB3, EPHA2, GJA1, PER2, FOXO1A, TGFBR3, CLU, ROR2, ETS2, TP73L, DDR2, BNIP2, FOXF1, MYO6, ABCC4, CRYAB, CYP27A1, FGF2, IKL, PTGIS, RARRES2, PLP2, TPM2, S100A6, SCHIP1, GOLPH2, TRIM36, POLD2, CGREF1, and HSD17B4.

In addition, the present invention relates, but is not limited to, kits for performing the aforementioned methods, as well as the uses for said kits.

### DESCRIPTION OF THE FIGURES

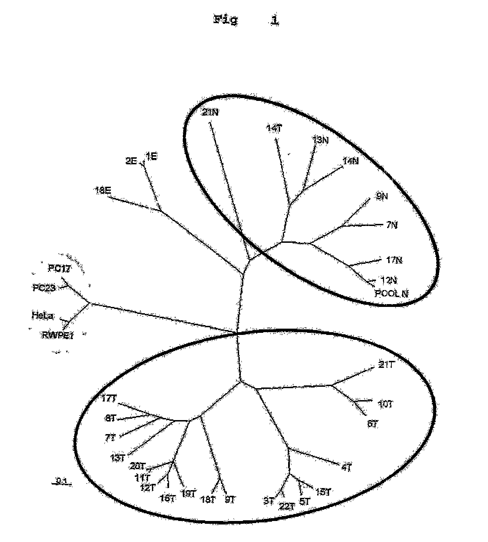

**Figure 1****.** Clusters of samples analyzed on HGF (Human Genome Focus) arrays by means of FADA [13]. Samples were clustered automatically into carcinomatous (circle in the lower part of the Figure), normal (circle at top-right of the Figure), cell lines (circle on the left of the Figure), and stromal samples (circle at top left of the Figure).

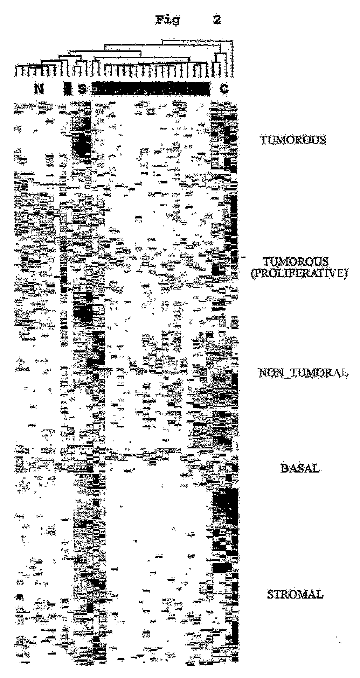

**Figure 2****.** Eisen representation, after analysis by FADA and hierarchical clustering (HC), of the 318 genes over- and underexpressed in prostate samples that differentiate more significantly between normal prostate tissue samples and samples of carcinomatous prostate (Table 2). The expression values used to generate the hierarchical clusters are those corresponding to Table 6. The hierarchy is established by the so-called hierarchical clustering method. It is a standard method used in applied statistics and therefore any person skilled in the art can derive the result obtained in the present invention from the numerical values in Table 6. In the upper part of the image: N, samples of normal prostate; T, samples of prostatic adenocarcinoma; S, samples of pure prostatic stroma; C, culture cells. On the right are indicated the compartments to which the different groups of genes predominantly correspond. This is a *post hoc* interpretation, i.e. arrived at on the basis of the expression profiles observed for these genes.

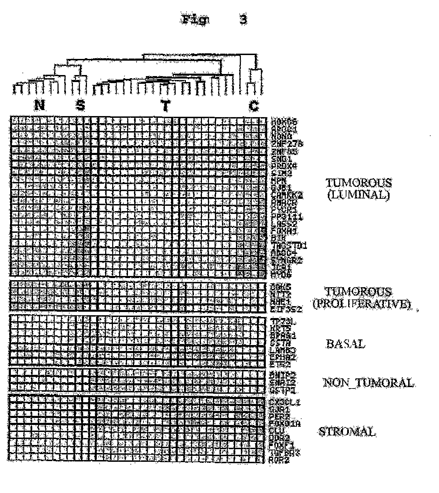

**Figure 3****.** Hierarchical clustering of the 30 samples analyzed on Affymetrix HGF arrays, using the 45 genes included on Diagnostic Chip 1 (Table 3). The expression values used to generate the hierarchical clusters are those corresponding to Table 6. The resulting sample clusters are denoted as described for Figure 2: N, normal prostate tissue; T, carcinomatous tissue; S, stroma; C, culture cells.

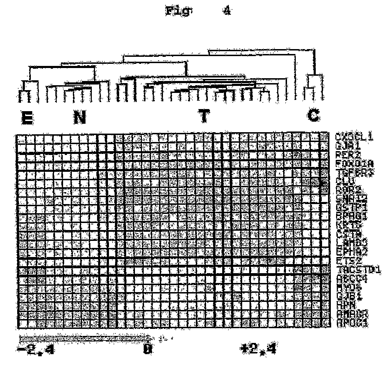

**Figure 4****.** Eisen diagram corresponding to the analysis by hierarchical clustering of the expression patterns in carcinomatous prostate, normal prostate, pure prostatic stroma, and cell lines, obtained on Affymetrix HGF arrays from 22 genes selected and validated by real-time RT-PCR (Table 4). The values used for the hierarchical clustering of these 22 genes were taken from Table 6. The resulting sample clusters are denoted as described for Figure 2: N, normal prostate tissue; T, carcinomatous tissue; S, stroma; C, culture cells.

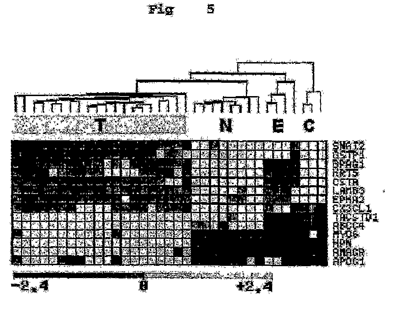

**Figure 5****.** Eisen diagram corresponding to the analysis by hierarchical clustering of the expression patterns in carcinomatous prostate, normal prostate, pure prostatic stroma, and cell lines, obtained on Affymetrix HGF arrays from 14 genes selected and validated by real-time RT-PCR (Table 5). The values used for the hierarchical clustering of these 14 genes were taken from Table 6. The resulting sample clusters are denoted as described for Figure 2: N, normal prostate tissue; T, carcinomatous tissue; S, stroma; C, culture cells.

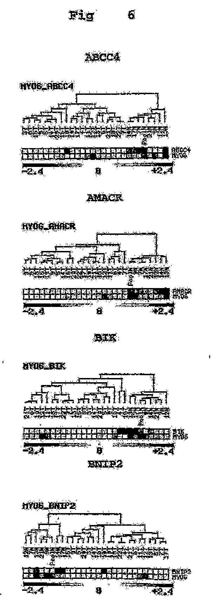

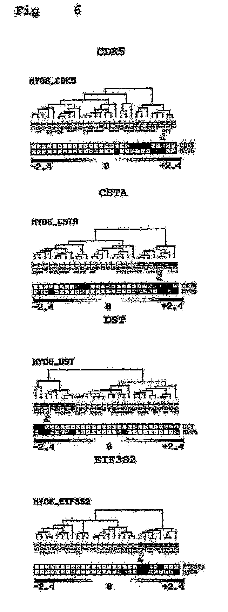

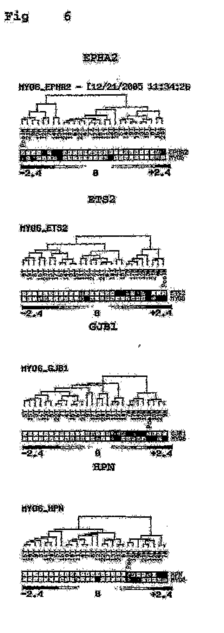

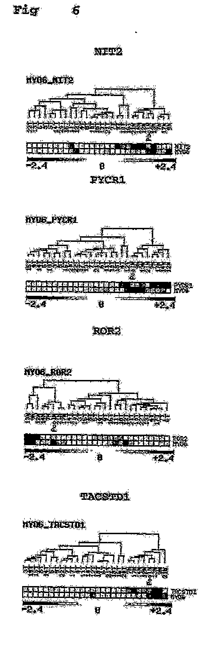

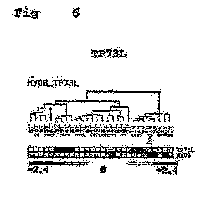

**Figure 6****.** Differentiation between samples of carcinomatous prostate (T) and normal prostate (N) by determining transcription levels using Affymetrix arrays of the MYO6 gene in combination with determining the transcription levels of the following genes: ABCC4, AMACR, BIK, BNIP2, CDK5, CSTA, DST, EIF3S2, EPHA2, ETS2, GJB1, HPN, NIT2, PYCR1, ROR2, TACSTD1, and TP73L. The expression values used for the hierarchical clustering of these genes were taken from Table 6.

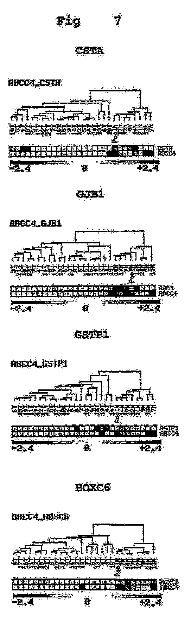

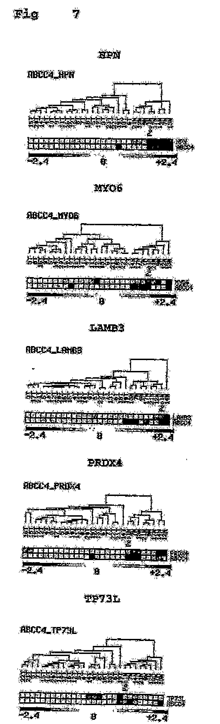

**Figure 7****.** Discrimination between samples of carcinomatous prostate (T) and normal prostate (N) by determining transcript levels with Affymetrix arrays of the ABCC4 gene in combination with determining the transcription levels of the following genes: CSTA, GJB1, GSTP1, HOXC6, HPN, LAMB3, MYO6, PRDX4, and TP73L. The expression values used for the hierarchical clustering of these genes were taken from Table 6.

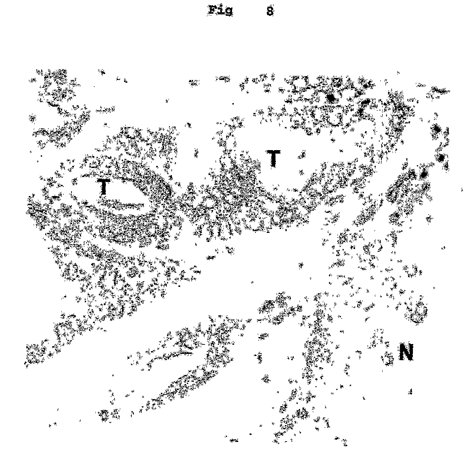

**Figure 8****.** Immunohistochemical detection of MYO6 protein in a tissue sample from a prostate cancer patient containing carcinomatous glands (T) and normal glands (N). The staining is clearly more intense in the carcinomatous epithelial cells than in the normal epithelial cells. The staining for MYO6 exhibits a cytoplasmic pattern having submembranous reinforcement.

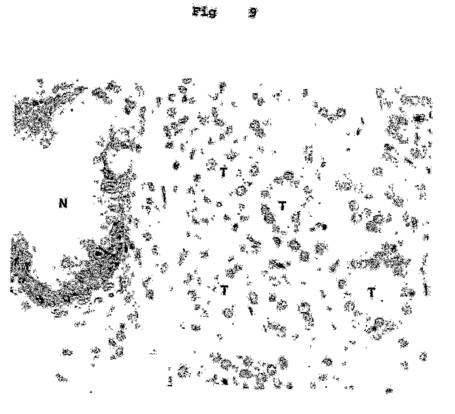

**Figure 9****.** Immunohistochemical detection of EPHA2 protein in a tissue sample from a prostate cancer patient containing carcinomatous glands (T) and normal glands (N). The staining is exclusively in normal glands, specifically in basal layer cells. The staining exhibits a cytoplasmic pattern having membranous reinforcement.

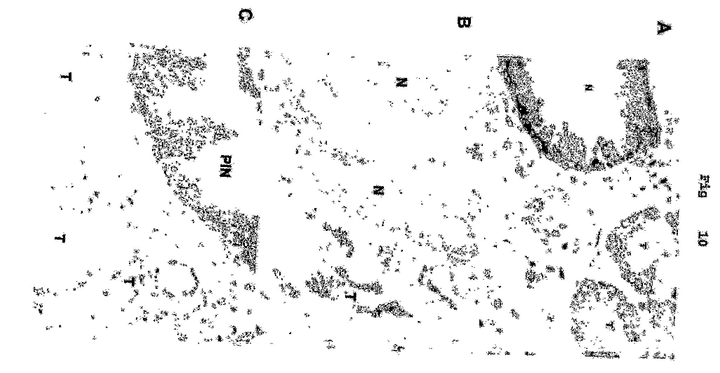

**Figure 10****.** Immunohistochemical detection of CX3CL1 protein in various prostate tissue samples. Figure 10 A: Sample comprising carcinomatous glands (T) and normal glands (N), wherein the CX3CL1 levels are clearly lower in carcinomatous cells than in normal cells. Figure 10 B: Samples comprising carcinomatous epithelial cells, wherein the staining for CX3CL1 is intense in most of the carcinomatous cells. Figure 10 C: Sample with PIN (P) and normal glands (N), wherein the staining is significantly more intense in the PIN cells than in the normal epithelial cells.

### Detailed description of the invention

For the realization of the present invention, a total of 31 prostate samples were analyzed by hybridization on Affymetrix Human Genome Focus arrays (Figure 1).

The raw hybridization signals were normalized by the method of Irizarry *et al.* (2003) and subjected to unsupervised analysis using the FADA algorithm [13]. Genes were considered to be differentially expressed between normal and carcinomatous groups when their associated q-value [17] was less than 2.5 × 10⁻⁴. This analysis allowed samples to be clustered automatically, such that all the cancer samples, except one, were clustered in one clade and all the normal samples were clustered in another clade (Figure 1). Established prostate cell lines and cells from primary explants obtained from samples of human prostate were also included in this analysis. In the FADA analysis, the cultured cells were clustered separately from the 2 aforementioned clades. From this analysis it was possible to deduce which genes are able to differentiate with the highest statistical significance (with p ≤ 10⁻⁴ in Student's t-test with multiple correction) between carcinomatous samples and normal samples; a total of 318 genes were identified in this way, whereof 134 were found to be significantly over-represented (overexpressed) in cancers and 184 significantly under-represented in cancers (Table 2 gives a list of genes capable of differentiating between samples of carcinomatous prostate and normal prostate, analyzed according to their expression profiles obtained by hybridization on Affymetrix HGF microarrays).

Some of these genes and their relevance in PC are described in rather greater detail in the following chapters.

The previously studied genes overexpressed in PC (Figure 2) were analyzed first. The identification of these genes served as external validation for the study. Genes in this category include the much investigated HPN and AMACR and, to a lesser extent, genes such as SIM2 and HOXC6. HPN has been extensively characterized [19-21], and it has been shown very recently that its overexpression can lead to a transformed phenotype in mouse models of prostate cancer [22]. AMACR has also been studied in many laboratories as a malignity marker in PC [23-27], and its clinical use has recently been expanded [26-29]. SIM2 has also been found, to a more limited extent, associated with PC [29], though it has already been studied as a possible therapy target with siRNA and antisense oligonucleotides in cell models [29, 31]. HOXC6 has been studied both as a malignity marker [32-35] and in its role in the survival of cultured prostate cancer cells [36].

Next, genes overexpressed in PC were analyzed that had not previously been unequivocally associated with prostate cancer. Among these genes there are many that appear in "lists" of genes from studies using microarray analysis, but none of these studies place any special emphasis on their biological characterization or make any special efforts in that direction. Among these genes there are transcription factors of very great interest in this context (FOXA1, NONO, ZNF278, ZNF85), vesicle transport protein genes (MYO6, RAB17, SYNGR2, RABIF), membrane transport genes (ABCC4, TMEM4, SLC19A7), fatty acid metabolism and nucleic acid metabolism-related enzyme genes.

The third group to be analyzed corresponded to the genes underexpressed in PC. Among the 184 genes detected as being significantly underexpressed in cancers, there is a relatively large number of genes that are expressed in stromal cells, so that it is suspected that, despite the care taken in selecting the samples to ensure a balance of the stromal component in carcinomatous and normal samples, the stromal component is more strongly represented in normal samples. However, there are also a large number of genes that appear to be typical of normal prostate epithelium and which are the ones that allow unsupervised clustering of normal samples in one and the same phyletic branch, separated from the stromal samples (Figure 1). Some of these genes have already been described as exhibiting decreased expression in PC. Two examples are GSTP1 and LOH11CR2A, and it has already been shown that the absence of expression in tumors of these two genes is due to CpG island hypermethylation in their promoter regions [37, 38]. Another interesting gene is TP73L, which codes for p63 and of which several isoforms (principally ΔN and TA) are involved in the effector function of the p53 cancer suppressor gene [39]. It has been shown, in addition, that p63 expression is associated with basal epithelial cells of the normal prostate gland, and that deletion of this gene in mice impedes the formation of a normal prostate [40].

Furthermore, primary cultures of prostate epithelial cells, as well as prostate cell lines immortalized with HPV-16, but not tumorigenic ones (e.g. RWPE1), express TP73L, while prostate cells established from tumors do not express this gene. Other underexpressed genes in cancers are transcription factors of the FOX family (FOXO1A, FOXF1) and other transcription factors, potential cancer suppressors (TACC1, SLIT2), transmembrane receptors and their ligands (TGFBR3, TGB3, FGFR1, FGF2, FGF7, IL6R), or cell adhesion proteins (DDR2, CADH9, ITGA5, GJA1).

Additionally, the expression levels of some of the proteins corresponding to genes overexpressed or underexpressed in PC in the present study as well as in previous studies [13] were validated by immunohistochemistry on paraffin-embedded samples (in Tissue Microarray format).

One of the genes found overexpressed in the transcription studies, and whose protein was studied by immunohistochemistry, was MY06. The present immunohistochemical study validated the transcription data, showing that the MYO6 protein is also overexpressed in the majority of cancers. A clear example of overexpression of the MYO6 protein in prostate cancer, by comparison with normal prostate glands, is shown in Figure 8, which corresponds to a sample containing both carcinomatous prostate epithelium and normal prostate epithelium, having been stained with an MYO6-specific monoclonal antibody. This protein is an atypical myosin with endocytosis and vesicular transport functions and which previously had been shown to be expressed in large amounts in ovarian cancer, principally in association with invasive edges [41].

An analysis was also conducted of the *in situ* expression of several of the genes underexpressed in the present invention, in particular those whose underexpression represent a novelty in this neoplasia, such as the tyrosine kinase receptor EPHA2, the transcription regulator SNAI2, or the chemokine CX3CL1. These results are worth highlighting, especially in relation to EPHA2 and SNAI2, as both EPHA2 [42-58] and SNAI2 [59-62] have been associated in numerous publications with overexpression rather than underexpression in many types of cancer, including prostate adenocarcinoma.

An example of the absence of EPHA2 protein expression in carcinomatous prostate epithelium is shown in Figure 9, wherein it is observed that while the normal prostate glands (in cells of the basal layer) express high levels of EPHA2, the adjacent carcinomatous prostate epithelial cells completely lack any reactivity and therefore express no detectable levels of this protein.

In the case of the CX3CL1 chemokine (also called fractalkine), the expression determined by real-time RT-PCR indicated a tendency for the carcinomatous epithelium to exhibit lower expression levels than the normal epithelium. Immunohistochemical staining for the corresponding protein, however, revealed variable profiles depending on the case, so that in some samples there was a significant decrease in CXC3L1 expression in carcinomatous epithelium, while in other cases the carcinomatous prostate epithelium gave high levels of said protein (Figure 10). Finally, various cases of prostatic intraepithelial neoplasia (PIN) showed variable levels of staining for CX3CL1, being in some cases of greater intensity than in the adjacent normal epithelium (Figure 10).

In the context of PC, therefore, our results indicate that, contrary to what has been generally accepted, the possible overexpression of these molecules should not be used as an indicator of malignity or serve as a therapeutic target in cancers of this type. Our data indicate, in fact, that the level of expression of these molecules in malignant prostate epithelium is low or nonexistent.

As a consequence of the foregoing analyses, a set of genes has been identified and defined, corresponding to the group of 318 genes, and also several subsets of genes on the basis of the former, useful for the molecular diagnosis of prostate cancer and having a high capacity for differentiating between carcinomatous and noncarcinomatous samples, wherein the determination of the levels of mRNA and/or protein represents a diagnostic signature of prostate cancer that constitutes a significant improvement over existing methods for the diagnosis of said cancer.

With the aim of designing a method for the diagnosis of prostate cancer in a format that is smaller than the set of 318 genes, more practical, and more akin to clinical practice (e.g. by means of RT-PCR analysis on a microarray or diagnostic chip), a smaller group of genes included in this first set was selected (see Example 2). This selection of a subset of 60 genes represents one of the many alternatives that can be obtained from the analysis of the original group of genes and should not be regarded as limiting the scope of the present invention. A person skilled in the art could come up with groups of genes different from those described in the present invention.

The first of the subsets contains a carefully selected set of 45 genes, validated by real-time RT-PCR, having a high capacity to differentiate between normal and carcinomatous samples (Table 3, Figure 3). Another generated version, for the analysis of a still smaller number of genes, validated by real-time RT-PCR, retains virtually the same capacity to differentiate between normal and carcinomatous samples as the foregoing. Said subset of genes included in this design corresponds to the 22 validated genes shown in Table 4 and Figure 4, or an even smaller subset of genes that corresponds to the 14 genes shown in Table 5 and Figure 5. Other generated versions of gene subsets having a high capacity to differentiate between carcinomatous and noncarcinomatous samples and falling within the scope of the present invention are shown in Figures 6 and 7. The identification of the expression levels of all these gene subsets serves as the basis for the development of a relatively low-cost and high-performance prostate cancer diagnostic kit or device for quantifying multiple transcripts, in real time, on a platform that allows a diverse and high number of samples to be analyzed simultaneously. Preferably, when the diagnostic kit is based on the quantitation of transcripts, less than 1 ng of total RNA is required per sample. It is equally possible to develop a prostate cancer diagnostic kit or device based on the determination of the protein levels of said genes in cancer samples.

Therefore, in an initial aspect, the present invention relates, but is not limited to, a method for the molecular diagnosis of prostate cancer comprising the *in vitro* analysis, in a test sample, of the expression level of at least one gene selected from the group of 60 genes consisting of: TACSTD1, HPN, AMACR, APOC1, GJB1, PP3111, CAMKK2, ZNF85, SND1, NONO, ICA1, PYCR1, ZNF278, BIK, HOXC6, CDK5, LASS2, NME1, PRDX4, SYNGR2, SIM2, EIF3S2, NIT2, FOXA1, CX3CL1, SNAI2, GSTP1, DST, KRT5, CSTA, LAMB3, EPHA2, GJA1, PER2, FOXO1A, TGFBR3, CLU, ROR2, ETS2, TP73L, DDR2, BNIP2, FOXF1, MYO6, ABCC4, CRYAB, CYP27A1, FGF2, IKL, PTGIS, RARRES2, PLP2, TPM2, S100A6, SCHIP1, GOLPH2, TRIM36, POLD2, CGREF1, and HSD17B4.

In another aspect, the present invention relates, but is not limited to, a method for the molecular diagnosis of prostate cancer comprising the *in vitro* analysis, in a test sample, of the expression level of at least two genes selected from the group of 60 genes consisting of: TACSTD1, HPN, AMACR, APOC1, GJB1, PP3111, CAMKK2, ZNF85, SND1, NONO, ICA1, PYCR1, ZNF278, BIK, HOXC6, CDK5, LASS2, NME1, PRDX4, SYNGR2, SIM2, EIF3S2, NIT2, FOXA1, CX3CL1, SNAI2, GSTP1, DST, KRT5, CSTA, LAMB3, EPHA2, GJA1, PER2, FOXO1A, TGFBR3, CLU, ROR2, ETS2, TP73L, DDR2, BNIP2, FOXF1, MYO6, ABCC4, CRYAB, CYP27A1, FGF2, IKL, PTGIS, RARRES2, PLP2, TPM2, S100A6, SCHIP1, GOLPH2, TRIM36, POLD2, CGREF1, and HSD17B4, wherein the capacity to discriminate between carcinomatous and noncarcinomatous samples when the expression levels of two or more genes from said group are determined together is greater than the discriminating capacity of the same genes separately.

In particular, the discriminating capacity when the expression levels of two or more genes are determined together is 1%, preferably 10%, more preferably 25%, more preferably still 50% greater than the differentiating capacity of at least one of the genes separately.

In the context of the present invention, "discriminating capacity" is defined as the capacity to discriminate between carcinomatous and noncarcinomatous samples when applying a method for classifying samples based on the set of data obtained from expression analysis experiments for one gene or for a subset of at least two genes from the group of 60 genes that is the object of the present invention.

For example, when applying a given classification method to the set of samples described in Table 6, the capacity of the genes MYO6 and CDK5 to discriminate between carcinomatous and noncarcinomatous samples determined individually was 93.6% and 87.1%, respectively, whereas the discriminating capacity of both genes determined together was 96.8%. In another example, the discriminating capacity of the genes ABCC4 and FOXO1A determined individually was 87.1% and 83.9%, respectively, whereas the discriminating capacity of both genes determined together was 96.8%.

The expression "test sample" as used in the description refers, but is not limited to, biological tissues and/or fluids (blood, urine, saliva, etc.) obtained by means of biopsies, curettage, or any other known method serving the same purpose and performed by a person skilled in the art, from a vertebrate liable to have prostate cancer, where said vertebrate is a human.

In a preferred embodiment, the present invention relates, but is not limited to, a method for the molecular diagnosis of prostate cancer comprising the *in vitro* analysis, in a test sample, of the expression level of at least two genes selected from the group of 22 genes consisting of: TACSTD1, HPN, AMACR, APOC1, GJB1, CX3CL1, SNAI2, GSTP1, DST, KRT5, CSTA, LAMB3, EPHA2, GJA1, PER2, FOXO1A, TGFBR3, CLU, ROR2, ETS2, MYO6, and ABCC4, wherein the capacity to discriminate between carcinomatous and noncarcinomatous samples when the expression levels of two or more genes from said group are determined together is greater than the discriminating capacity of the same genes separately.

In another preferred embodiment, the present invention relates, but is not limited to, a method for the molecular diagnosis of prostate cancer comprising the *in vitro* analysis, in a test sample, of the expression level of at least two genes selected from the group of 14 genes consisting of: TACSTD1, HPN, AMACR, APOC1, CX3CL1, SNAI2, GSTP1, KRT5, DST, LAMB3, CSTA, EPHA2, MY06, and ABCC4, wherein the capacity to discriminate between carcinomatous and noncarcinomatous samples when the expression levels of two or more genes from said group are determined together is greater than the discriminating capacity of the same genes separately.

In another preferred embodiment, the present invention relates, but is not limited to, a method for the molecular diagnosis of prostate cancer comprising the *in vitro* analysis, in a test sample, of the expression level of at least two genes selected from the group of 7 genes consisting of: TACSTD1, HPN, DST, CSTA, LAMB3, EPHA2, and MY06, wherein the capacity to discriminate between carcinomatous and noncarcinomatous samples when the expression levels of two or more genes from said group are determined together is greater than the discriminating capacity of the same genes separately.

In a third aspect, the present invention relates, but is not limited to, a method for the molecular diagnosis of prostate cancer having a high capacity to discriminate between carcinomatous and noncarcinomatous samples, comprising the *in vitro* analysis, in a test sample, of the expression level of at least two genes selected from Table 3, wherein at least one of said selected genes is MY06 or ABCC4.

In a preferred embodiment, the present invention relates, but is not limited to, a method for the molecular diagnosis of prostate cancer having a high capacity to discriminate between carcinomatous and noncarcinomatous samples, comprising the *in vitro* analysis, in a test sample, of the expression level of the MYO6 gene in combination with the analysis of the expression level of at least one gene from the group consisting of: ABCC4, AMACR, BIK, BNIP2, CDK5, CSTA, DST, EIF3S2, EPHA2, ETS2, GJB1, HPN, NIT2, PYCR1, ROR2, TACSTD1, and TP73L.

In a still more preferred embodiment, the present invention relates, but is not limited to, a method for the molecular diagnosis of prostate cancer with a high capacity to discriminate between carcinomatous and noncarcinomatous samples, comprising the *in vitro* analysis, in a test sample, of the overexpression of the MYO6 gene in combination with the analysis of the overexpression of at least one gene from the group consisting of: ABCC4, AMACR, BIK, CDK5, EIF3S2, GJB1, HPN, NIT2, PYCR1, and TACSTD1.

In a still more preferred embodiment, the present invention relates, but is not limited to, a method for the molecular diagnosis of prostate cancer having a high capacity to discriminate between carcinomatous and noncarcinomatous samples, comprising the *in vitro* analysis, in a test sample, of the overexpression of the MYO6 gene in combination with the analysis of the underexpression of at least one gene from the group consisting of: BNIP2, CSTA, DST, EPHA2, ETS2, ROR2, and TP73L.

In another preferred embodiment, the present invention relates, but is not limited to, a method for the molecular diagnosis of prostate cancer with a high capacity to differentiate between carcinomatous and noncarcinomatous samples, comprising the *in vitro* analysis, in a test sample, of the expression level of the ABCC4 gene in combination with the analysis of the expression level of at least one gene from the group consisting of: CSTA, GJB1, GSTP1, HOXC6, HPN, LAMB3, MY06, PRDX4, and TP73L.

In a still more preferred embodiment, the present invention relates, but is not limited to, a method for the molecular diagnosis of prostate cancer having a high capacity to discriminate between carcinomatous and noncarcinomatous samples, comprising the *in vitro* analysis, in a test sample, of the overexpression of the ABCC4 gene in combination with the analysis of the overexpression of at least one gene from the group consisting of: GJB1, HOXC6, HPN, MYO6 and PRDX4.

In a still more preferred embodiment, the present invention relates, but is not limited to, a method for the molecular diagnosis of prostate cancer having a high capacity to differentiate between carcinomatous and noncarcinomatous samples, comprising the *in vitro* analysis, in a test sample, of the overexpression of the ABCC4 gene in combination with the analysis of the underexpression of at least one gene from the group consisting of: CSTA, GSTP1, LAMB3, and TP73L.

In another preferred embodiment, the present invention relates, but is not limited to, a method for the molecular diagnosis of prostate cancer having a high capacity to differentiate between carcinomatous and noncarcinomatous samples, comprising the *in vitro* analysis, in a test sample, of the overexpression of MYO6, TACSTD1, or HPN genes or the analysis of the underexpression of DST, CSTA, LAMB3, or EPHA2 genes.

In another preferred embodiment, the present invention relates, but is not limited to, a method for the molecular diagnosis of prostate cancer having a high capacity to differentiate between carcinomatous and noncarcinomatous samples, comprising the *in vitro* analysis, in a test sample, of the overexpression of MYO6, ABCC4, TACSTD1, HPN AMACR, or APOC1 genes or the analysis of the underexpression of the CX3CL1, SNAI2, GSTP1, DST, KRT5, CSTA, LAMB3, or EPHA2 genes.

In a still more preferred embodiment, the present invention relates, but is not limited to, a method for the molecular diagnosis of prostate cancer having a high capacity to differentiate between carcinomatous and noncarcinomatous samples, comprising the *in vitro* analysis, in a test sample, of the overexpression of MYO6, ABCC4, TACSTD1, HPN, AMACR, APOC1, or GJB1, or analysis of the underexpression of genes CX3CL1, SNAI2, GSTP1, DST, KRT5, CSTA, LAMB3, EPHA2, GJA1, PER2, FOXO1A, TGFBR3, CLU, ROR2, or ETS2.

In a still more preferred embodiment, the present invention relates, but is not limited to, a method for the molecular diagnosis of prostate cancer having a high capacity to differentiate between carcinomatous and noncarcinomatous samples, comprising the *in vitro* analysis, in a test sample, of the overexpression of MYO6, ABCC4, TACSTD1, HPN, AMACR, APOC1, GJB1, PP3111, CAMKK2, ZNF85, SND1, NONO, ICA1, PYCR1, ZNF278, BIK, HOXC6, CDK5, LASS2, NME1, PRDX4, SYNGR2, SIM2, EIF3S2, NIT2, FOXA1, GOLPH2, TRIM36, POLD2, CGREF1, or HSD17B4, or analysis of the underexpression of genes PRDX4 CX3CL1, SNAI2, GSTP1, DST, KRT5, CSTA, LAMB3, EPHA2, GJA1, PER2, FOXO1A, TGFBR3, CLU, ROR2, ETS2, TP73L, DDR2, BNIP2, FOXF1, CRYAB, CYP27A1, FGF2, IKL, PTGIS, RARRES2, PLP2, TPM2, S100A6, or SCHIP1.

"Overexpressed gene" as used in the present invention should be understood to mean, in general, the abnormally high expression of a gene or of its transcription or expression products (RNA or protein) in cells coming from tumorigenic prostate tissue, when compared to the expression of said gene or its transcription or expression products (RNA or protein) in normal cells of the same nontumorigenic tissue. In the case of determining expression levels by hybridization on Affymetrix microarrays, any gene in a prostate cancer sample whose expression levels are at least 2.0 times as high as the expression levels of the corresponding noncarcinomatous prostate tissue sample is defined as "overexpressed". When the determination is performed by quantitative RT-PCR, the term "overexpression" applies when the expression level of the gene in question in the cancer sample is at least 1.5 times the expression level in the corresponding normal prostate sample. However, when several cancer samples are being analyzed, a gene is considered to be "generally overexpressed" or "overexpressed in such prostate cancers when said gene is overexpressed in at least 70% of the cancer samples studied, comparing the normalized levels of said gene, determined in carcinomatous prostate tissue samples, with the arithmetic mean of the normalized levels of at least five samples of noncarcinomatous prostate tissue, the "overexpression" levels being quantitatively defined as described above for determinations on microarrays or by quantitative RT-PCR.

"Underexpressed gene" as used in the present invention should be understood to mean, in general, the abnormally low expression of a gene or of its transcription or expression products (RNA or protein) in cells coming from tumorigenic prostate tissue, when compared to the expression of said gene or its transcription or expression products (RNA or protein) in normal cells of the same nontumorigenic tissue. In the case of determining expression levels by hybridization on Affymetrix microarrays, any gene in a prostate cancer sample whose expression levels are one-half or less of the expression levels of the corresponding noncarcinomatous prostate tissue sample is defined as "underexpressed." When the determination is performed by quantitative RT-PCR, the term "underexpression" applies when the expression level of the gene in question in the cancer sample is 0.75 times or less the expression level in the corresponding normal prostate sample. However, when several cancer samples are being analyzed, a gene is considered to be "generally underexpressed" or "underexpressed" in such prostate cancers when said gene is underexpressed in at least 70% of the cancer samples studied, comparing the normalized levels of said gene, determined in carcinomatous prostate tissue samples, with the arithmetic mean of the normalized levels of at least five samples of noncarcinomatous prostate tissue, the "underexpression" levels being quantitatively defined as described above for determinations on microarrays or by quantitative RT-PCR.

It was considered that a sample exhibited overexpression or underexpression of a protein with respect to another sample when the percentage difference in epithelial staining between the two samples was greater than 20% and/or the intensity differed by at least one point.

And, finally, in a still more preferred embodiment, the present invention relates, but is not limited to, a method for the molecular diagnosis of prostate cancer having a high capacity to discriminate between carcinomatous and noncarcinomatous samples, comprising the *in vitro* analysis, in a test sample, of the overexpression or underexpression of the 318 genes indicated in Table 2.

In a fourth aspect, the present invention relates, but is not limited to, a method for the molecular diagnosis of prostate cancer having a high capacity to differentiate between carcinomatous and noncarcinomatous samples, comprising the *in vitro* analysis, in a test sample, of the expression level of at least one gene or subsets of two genes selected from Table 3, wherein the analysis of the expression level of said genes is performed by determining the level of mRNA derived from their transcription and/or by determining the level of protein encoded by the gene or fragments thereof.

In a preferred embodiment, the present invention relates, but is not limited to, a method for the molecular diagnosis of prostate cancer having a high capacity to discriminate between carcinomatous and noncarcinomatous samples, comprising the *in vitro* analysis, in a test sample, of the expression level of at least one gene or subsets of two genes selected from Table 3, wherein the analysis of the expression level of said genes is performed by determining the level of mRNA derived from their transcription where the analysis of the mRNA level can be performed, by way of illustration and without limiting the scope of the invention, by PCR (polymerase chain reaction) amplification, RT-PCR (retrotranscription in combination with polymerase chain reaction), RT-LCR (retrotranscription in combination with ligase chain reaction), SDA, or any other nucleic acid amplification method; DNA chips produced with oligonucleotides deposited by any mechanism; DNA chips produced with oligonucleotides synthesized *in situ* by photolithography or by any other mechanism; *in situ* hybridization using specific probes labeled by any labeling method; by gel electrophoresis; by membrane transfer and hybridization with a specific probe; by NMR or any other diagnostic imaging technique using paramagnetic nanoparticles or any other type of detectable nanoparticles functionalized with antibodies or by any other means.

In another preferred embodiment, the present invention relates, but is not limited to, a method for the molecular diagnosis of prostate cancer having a high capacity to discriminate between carcinomatous and noncarcinomatous samples, comprising the *in vitro* analysis, in a test sample, of the expression level of at least one gene or subsets of two genes selected from Table 3, wherein the determination of the expression level of said genes is performed by determining the level of protein encoded by the gene or fragments thereof, by incubation with a specific antibody (wherein the analysis is performed by Western blot and/or by immunohistochemistry); by gel electrophoresis; by protein chips; by ELISA or any other enzymatic method; by NMR or any other diagnostic imaging technique.

The term "antibody" as used in the present description includes monoclonal antibodies, polyclonal antibodies, recombinant antibody fragments, combibodies, Fab and scFv antibody fragments, as well as ligand binding domains.

In a fifth aspect, the present invention relates, but is not limited to, a prostate cancer molecular diagnostic kit. Said kit may comprise primers, probes, and all the reagents necessary to analyze the variation in the expression level of at least one gene or subset of two genes of any of the aforementioned methods. The kit can additionally include, without any kind of limitation, the use of buffers, polymerases, and cofactors to ensure optimal activity thereof, agents to prevent contamination, etc. Furthermore, the kit can include all the media and containers necessary for start-up and optimization.

Accordingly, another object of the present invention is a device for the molecular diagnosis of prostate cancer, hereinafter called 'diagnostic device of the invention,' which comprises the necessary elements for analyzing the variation in the expression levels of at least one gene or subsets of two genes of any of the foregoing methods.

A preferred embodiment of the present invention consists in a diagnostic device of the invention for the detection of mRNA expression levels using a technique, by way of illustration and without limiting the scope of the invention, belonging to the following group: Northern blot analysis, polymerase chain reaction (PCR), real-time retrotranscription in combination with polymerase chain reaction (RT-PCR), retrotranscription in combination with ligase chain reaction (RT-LCR), hybridization, or microarrays.

Another preferred embodiment of the invention consists in a diagnostic device of the invention for the detection of mRNA expression levels comprising, by way of illustration and without limiting the scope of the invention, a DNA microarray, a DNA gene chip, or a microelectronic DNA chip, including gene probes.

Another preferred embodiment of the invention consists in a diagnostic device of the invention for the detection of protein expression levels using a technique, by way of illustration and without limiting the scope of the invention, a DNA microarray, belonging to the following group: ELISA, Western blot, and a protein biochip or a microarray-type device that includes specific antibodies.

In a sixth aspect, the present invention relates, but is not limited to, a method for the molecular diagnosis of prostate cancer having a high capacity to discriminate between carcinomatous and noncarcinomatous samples, comprising the *in vitro* analysis, in a test sample, wherein the overexpression of the genes MYO6, ABCC4, TACSTD1, HPN, AMACR, APOC1, or analysis of the underexpression of the genes CX3CL1, SNAI2, GSTP1, DST, KRT5, CSTA, LAMBr, or EPHA2 is used for the diagnosis of the presence of prostate cancer or of a premalignant condition thereof, or for the prognosis of the progression of the prostate cancer or of a premalignant condition thereof, or for the prognosis of the risk of recurrence of said disease.

In a preferred embodiment, the present invention relates, but is not limited to, a method for the molecular diagnosis of prostate cancer having a high capacity to discriminate between carcinomatous and noncarcinomatous samples, comprising the *in vitro* analysis, in a test sample, wherein the overexpression of MYO6, ABCC4, TACSTD1, HPN, AMACR, APOC1, or GJB1, or analysis of the underexpression of the genes CX3CL1, SNAI2, GSTP1, DST, KRT5, CSTA, LAMB3, EPHA2, GJA1, PER2, FOXO1A, TGFBR3, CLU, ROR2, or ETS2 is used for the diagnosis of the presence of prostate cancer or of a premalignant condition thereof, or for the prognosis of the progression of the prostate cancer or of a premalignant condition thereof, or for the prognosis of the risk of recurrence of said disease.

In a still more preferred embodiment, the present invention relates, but is not limited to, a method for the molecular diagnosis of prostate cancer having a high capacity to discriminate between carcinomatous and noncarcinomatous samples, comprising the *in vitro* analysis, in a test sample, wherein overexpression of the genes MYO6, ABCC4, TACSTD1, HPN, AMACR, APOC1, GJB1, PP3111, CAMKK2, ZNF85, SND1, NONO, ICA1, PYCR1, ZNF278, BIK, HOXC6, CDK5, LASS2, NME1, PRDX4, SYNGR2, SIM2, EIF3S2, NIT2, or FOXA1, or analysis of the underexpression of the genes CX3CL1, SNAI2, GSTP1, DST, KRT5, CSTA, LAMB3, EPHA2, GJA1, PER2, FOXO1A, TGFBR3, CLU, ROR2, ETS2, TP73L, DDR2, BNIP2, or FOXF1 is used for the diagnosis of the presence of prostate cancer or of a premalignant condition thereof, or for the prognosis of the progression of the prostate cancer or of a premalignant condition thereof, or for the prognosis of the risk of recurrence of said disease.

In a still more preferred embodiment, the present invention relates, but is not limited to, a method for the molecular diagnosis of prostate cancer having a high capacity to discriminate between carcinomatous and noncarcinomatous samples, comprising the *in vitro* analysis, in a test sample, wherein overexpression of the 318 genes indicated in Table 2 is used for the diagnosis of the presence of prostate cancer or of a premalignant condition thereof, or for the prognosis of the progression of the prostate cancer or of a premalignant condition thereof, or for the prognosis of the risk of recurrence of said disease.

Unless otherwise defined, all technical and scientific terms used herein have the same meanings as those commonly understood by a person skilled in the art to which the invention belongs. Throughout the description and claims the word "comprises" and its variants do not seek to exclude other technical characteristics, components, or steps. To persons skilled in the art, other objects, advantages, and characteristics of the invention will be apparent, partly from the description and partly in the practice of the invention. The following examples and drawings are provided by way of illustration and do not be regarded as in any way limiting the present invention.

### EXAMPLES OF THE INVENTION

### Example 1 - Identification of the genes associated with a cluster identifying a prostate cancer tumor pattern.

For the realization of the present invention, a series of 31 human prostate samples were analyzed by hybridization on Affymetrix Human Genome Focus arrays (Figure 1):
I. 20 samples enriched with carcinomatous epithelium.
II. 7 samples enriched with normal epithelium (< 1% of cancer cells).
III. 1 sample comprising a group of 5 normal samples (POOL N).
IV. 3 samples consisting exclusively of stromal tissue.

The collected tissues were embedded in OCT, frozen in isopentane, and stored at -80°C. The samples were assessed histologically and selected for analysis in accordance with the following criteria: (a) minimum 90% of pure normal or carcinomatous epithelium in the normal and carcinomatous samples, respectively; (b) absence or minimal presence of foci of inflammation or atrophy. All the samples except three (one normal and two carcinomatous) come from the peripheral region, including the stroma samples. The estimated mean epithelial content in the carcinomatous samples was 70%, an average 90% of which exhibited neoplastic characteristics. The estimated mean epithelial content in normal samples was 40%, with no carcinomatous glands. The stroma samples contained less than 1% of epithelium. For extracting total RNA from the tissues, 20-30 cryosections were used, each 20 µm thick. To confirm the diagnosis and the quality of the samples, the first and last section of every sample was stained with hematoxylin-eosin. Table 1 describes the clinico-pathological characteristics corresponding to the samples used.

**Table 1. Clinico-pathological characteristics corresponding to the samples used in the study**

| STROMA SAMPLES | | |
|---|---|---|
| 1E | | |
| 2E | | |
| 18E | | |

| CARCINOMATOUS SAMPLES | GLEASON SCORE | GRADE |
|---|---|---|
| 3T | 8 | T2 |
| 4T | 7 | T3a |
| 5T | 7 | T3a |
| 6T | 7 | T3a |
| 7T | 6 | T2 |
| 8T | 9 | T3a |
| 9T | 9 | T3a |
| 10T | 7 | T3a |
| 11T | 7 | T3a |
| 12T | 6 | T3a |
| 13T | 7 | T2 |
| 14T | 7 | T2 |
| 15T | 5 | T2 |
| 16T | 7 | T3a |
| 17T | 9 | T2 |
| 18T | 7 | T2c |
| 19T | 8 | T2 |
| 20T | 6 | T2 |
| 21T | 7 | T3a |
| 22T | 7 | T3a |
| NORMAL SAMPLES | | |
| 7N | | |
| 9N | | |
| 12N | | |
| 13N | | |
| 14N | | |
| 17N | | |
| 21N | | |

| PRIMARY CULTURES | ORIGINAL SAMPLE | |
|---|---|---|
| PC17 | 17T | |
| PC23 | 23T | |

| | | |
|---|---|---|
| *Clinical and pathological staging according to the international TNM classification of prostate adenocarcinoma. | | |

The Gleason score goes from 2 to 10 and describes the aggressiveness of the cancer cells and, therefore, the likelihood of the tumor spreading. The lower the score, the lower the likelihood of the tumor spreading.

The cell lines HeLa and RWPE-1 (obtained from the American Type Culture Collection) were cultured in DMEM (PAA, Ontario, Canada) supplemented with 10% of serum (FBS) and KSFM (Gibco, Garlsbad, CA), respectively, with the aim of using them as controls. The primary cultures (PC17 and PC23) were derived from radical prostatectomies from patients having clinically localized prostate cancer, in which the adenocarcinoma had been detected macroscopically. The tissue explants were washed in PBS, ground, and cultured in KSFM (Gibco, Carlsbad, CA) supplemented with 5-α-dihydrotestosterone at a concentration of 10¹¹ M. After 4-5 weeks of culturing and two passes, the cultures were morphologically assessed to ensure absence of fibroblasts and used to obtain total RNA.

The tissue samples were laser-microdissected. 8 µm cryosections were mounted on plastic membrane-covered glass slides (PALM Mikrolaser Technology, Bernried, Germany), fixed for 3 minutes in 70% ethanol, stained with Mayer's hematoxylin (SIGMA, St. Louis, MO), dehydrated in a series of alcohols, left to dry for 10 minutes and stored at - 80°C until used. The samples were microdissected using the PALM MicroBeam system (PALM Mikrolaser Technology). Approximately 1.2 mm² of normal or carcinomatous epithelium was collected for each sample and estimated to be 99% homogeneous by microscopic visualization.

Total RNA from the tissue samples and cell lines was extracted using the RNeasy Mini Kit (Qiagen, Valencia, CA). Total RNA from the microdissected samples was extracted with the RNeasy Micro Kit (Qiagen). In all cases there was a DNase I digestion step (Qiagen), and the RNA quality and concentration was assessed with the 2100 Bioanalyzer (Agilent Technologies, Palo Alto, CA).

For the gene expression analysis by microarray hybridization, RNA was used that had been isolated from 7 samples of normal prostate tissue with its corresponding pair (i.e. same patient and same surgical resection) of carcinomatous prostate sample, one sample comprising a mixture of equal parts of the RNA extracted from 5 samples of normal prostate tissue (normal pool), 13 unpaired carcinomatous samples (i.e. without a corresponding sample of normal prostate tissue from the same patient), 3 samples of pure normal prostate stroma (without epithelial tissue), two established epithelial cell lines (HeLa and RWPE-1), and two primary prostate cultures (PC17 and PC23). cDNA was synthesized from 2 µg of total RNA, using a primer having a promoter sequence for RNA polymerase T7 added at the 3' end (Superscript II Reverse Transcriptase, Invitrogen, Carlsbad, CA). After synthesis of the second chain, an *in vitro* transcription was performed using the BioArray High Yield RNA Labeling Kit (Enzo, Farmingdale, NY) to obtain biotin-labeled cRNA.

Prior to hybridization, washing, and scanning of the microarrays, the cRNA (15 µg) were heated at 95°C for 35 min to provide fragments 35-200 bases long. Each sample was added to a hybridization solution [100 mM 2-(N-morpholino)ethanesulfonic acid, 1 M Na⁺, and 20 mM EDTA] in the presence of 0.01% Tween-20 at a final concentration of cRNA of 0.05 µg/mL. 5 µg of fragmented cRNA was hybridized on a TestChip (Test3, Affymetrix, Santa Clara, CA) by way of quality control. 10 µg of each fragmented cRNA were hybridized on Affymetrix Human Genome Focus Arrays at 45°C for 16 h, washed and stained in the Affymetrix Fluidics Station 400, and scanned at 3 µm resolution in an Agilent HP G2500A GeneArray scanner (Agilent Technologies, Palo Alto, CA).

Computer analysis was then performed and the results obtained were normalized. The raw hybridization signals were normalized in accordance with the normalization method described by Irizarry *et al.* using the RMA algorithm [14], available as part of the Bioconductor package from Affymetrix. The first step in the RMA normalization procedure is to subtract the background signal; this is achieved taking into account that the observed PM probes can be modeled as a signal component that follows a normal distribution. The distribution parameters are adjusted on the basis of the data and the noise component is then eliminated. Normalization between arrays is then performed by quantile-quantile normalization at probe level, using the method proposed by Bolstad *et al.* [15]. The goal is for all the chips to have the same empirical distribution. Finally, the observed intensities of the groups of probes are summarized to obtain the measurement of the expression of each gene using the median polish algorithm [16], which is adapted to this model in a robust manner.

Prior to selecting the differentially expressed genes and to modeling the gene networks or the groups of genotypically consistent samples (see below), the genotypic consistency of the samples belonging to each of the groups was checked. The normalized expression data were analyzed using the FADA program [13]. This program applies a Q-Mode Factor Analysis, a multivariate tool related to PCA, coupled to clustering algorithms in sample space. Genes were considered to be differentially expressed between the normal and carcinomatous groups when their associated q-value [17] was less than 2.5 × 10⁻⁴. The q-values were calculated from the p-values obtained from the t-test using the Benjamini-Hochberg step-down false-discovery rate (FDR) algorithm [18], as implemented in the Bioconductor multitest package. This algorithm adjusts the p-values upward to eliminate the effects of multiple testing.

In the context of the present invention it is understood that the values of a parameter discriminate between two classes or categories of samples (in our case, carcinomatous samples and normal samples) with high significance when the value of p in a statistical comparison (by applying e.g. the t-test) between the two categories is < 0.001.Table 6 shows the numerical data corresponding to the expression levels of the genes shown in the first column for the samples shown in the first row. Samples ending in T correspond to carcinomatous prostate and those ending in N correspond to normal prostate. Table 6 also shows the expression values for the cell lines HeLa (originating in a human cervical cancer) and RWPE-1 (human prostate epithelium transformed with the herpes virus HPV16), and for two primary explants derived from prostate cancers, designated PC17 and PC23. The digits are values of the signals obtained by hybridization of labeled cRNA on Affymetrix HGF microarrays, normalized by the MRA method [14].

This analysis enabled samples to be clustered automatically, such that all the carcinomatous samples, except one, were clustered in one clade and all the normal samples were clustered in another clade (Figure 1). At the same time, the cultured cells and the stroma samples were clustered separately from the 2 aforementioned clades (Figure 1).

From this analysis it was possible to identify the genes that were able to discriminate with the highest significance level (with p ≤ 10⁻⁴ in Student's t-test with multiple correction) between carcinomatous samples and normal samples; a total of 318 genes were identified in this way, whereof 134 were found to be significantly over-represented (overexpressed) in cancers and 184 significantly under-represented (underexpressed) in cancers (Table 2).

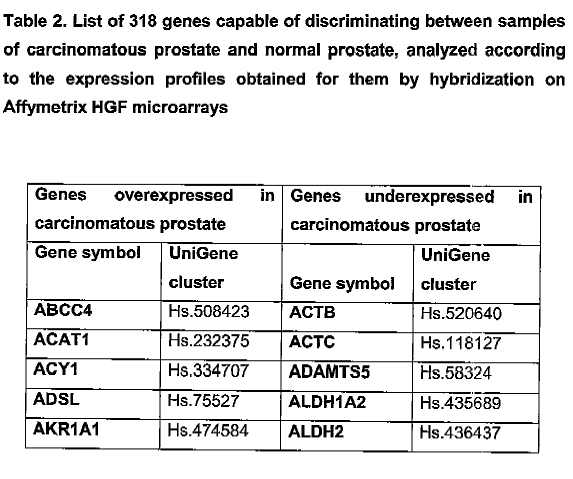

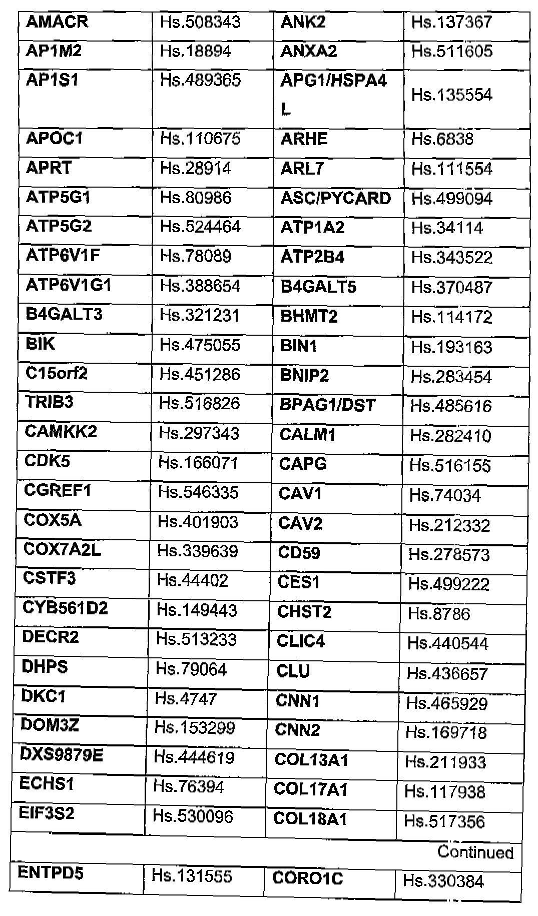

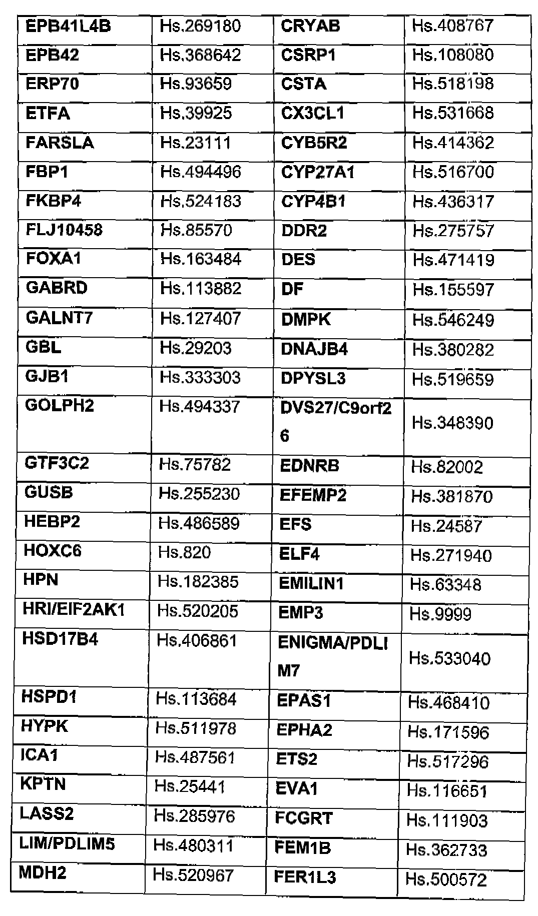

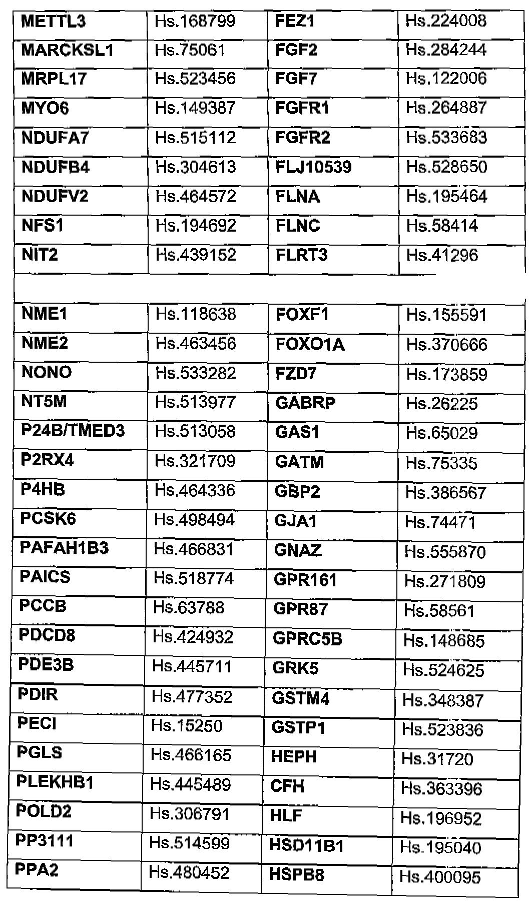

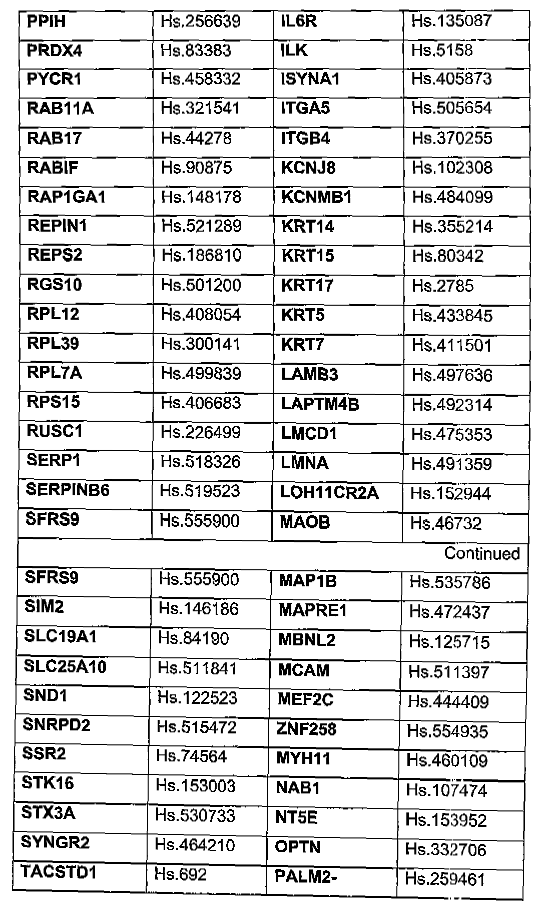

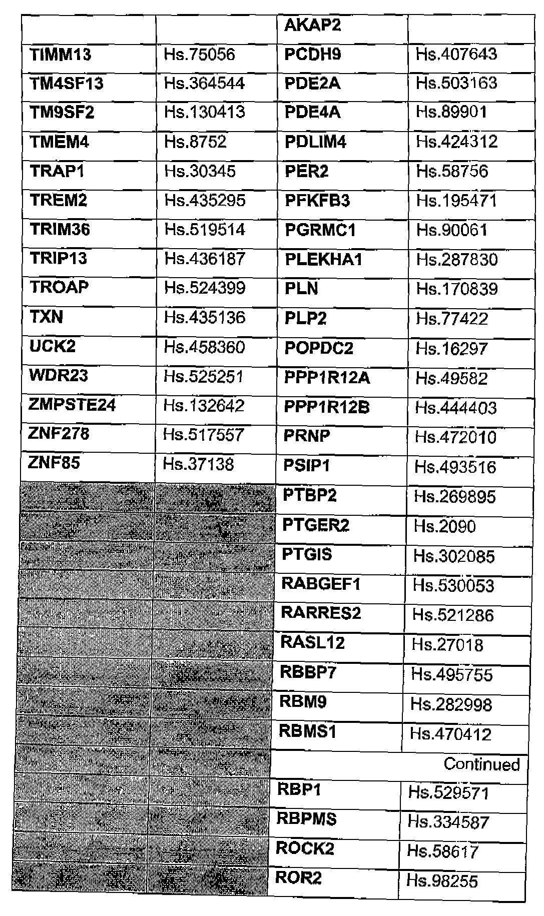

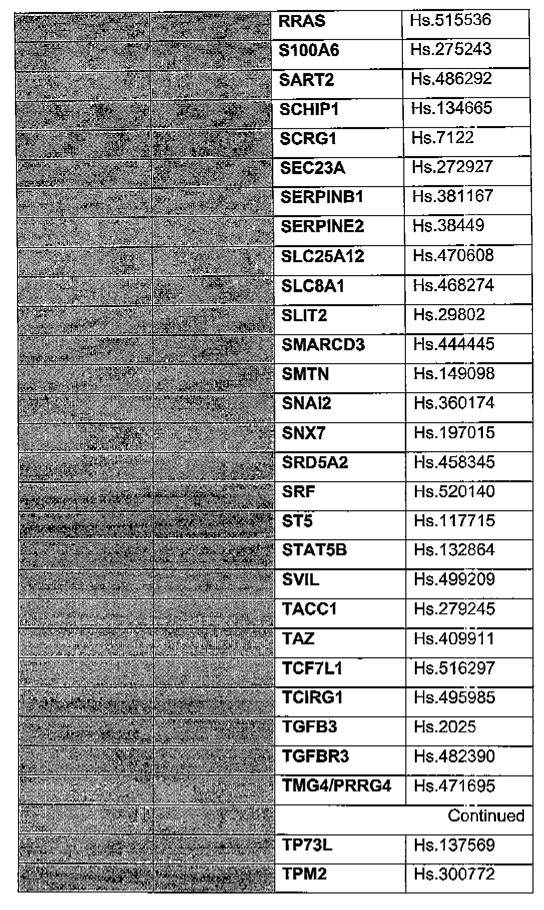

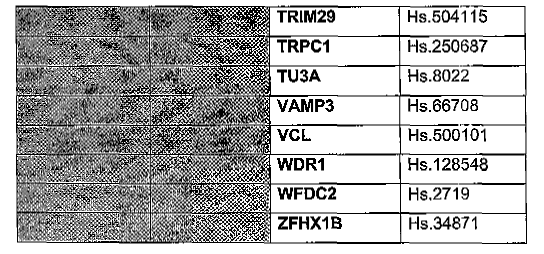

### Example 2 - Validation of the genes identified as most relevant in the microarray hybridization experiments by means of real-time RT-PCR using the TaqMan LDA format.

This was done by performing real-time RT-PCR on the genes of greatest interest biologically and as markers from the complete panel of 318 genes identified previously, using both non-microdissected samples and samples laser-microdissected using the PALM instrument. The object of the RT-PCR analysis is to determine the expression levels of these genes in a diagnostic chip-type format, which is smaller and more akin to clinical practice.

Real-time RT-PCR was carried out for each replica of prostate tissue (in triplicate) or of microdissected samples (in quadruplicate), whether of carcinomatous or normal tissue. Thus, 1 ng of starting total RNA was used for the synthesis of cDNA using the reverse transcriptase Superscript II (Invitrogen) and random hexamers at 42°C for 50 min, followed by treatment with RNase at 37°C for 20 min. The resulting cDNA were used to perform real-time PCR in an ABI PRISM 7900HT instrument (Applied Biosystems, Foster City, CA), using a specially designed TaqMan Low Density array (Applied Biosystems) containing primers and probes specific for 45 genes of interest and the RPS18 gene for calibration, and designated as Diagnostic Chip 1 (see Table 3). The Thermocycler conditions were established in accordance with the manufacturer's specifications. The data obtained were analyzed using the SDS 2.1 software (Applied Biosystems) applying the ΔΔCT relative quantification method.

**Table 3. List of the 45 genes that best discriminate between carcinomatous and normal prostate samples**

| **Genes overexpressed in carcinomatous prostate** | | **Genes underexpressed in carcinomatous prostate** | |
|---|---|---|---|
| **Gene symbol** | **UniGene cluster** | **Gene symbol** | **UniGene cluster** |
| **PP3111** | Hs.514599 | **DDR2** | Hs.275757 |
| **CAMKK2** | Hs.297343 | **CLU** | Hs.436657 |
| **ZNF85** | Hs.37138 | **TP73L** | Hs.137569 |
| **MY06** | Hs.149387 | **SNAI2** | Hs.360174 |
| **SND1** | Hs.122523 | **ETS2** | Hs.517296 |
| **NONO** | Hs.533282 | **KRT5** | Hs.433845 |
| **ICA1** | Hs.487561 | **TGFBR3** | Hs.482390 |
| **ABCC4** | Hs.508423 | **GSTP1** | Hs.523836 |
| **PYCR1** | Hs.458332 | **ROR2** | Hs.98255 |
| **ZNF278** | Hs.517557 | **LAMB3** | Hs.497636 |
| **TACSTD1** | Hs.692 | **BPAG1/DST** | Hs.485616 |
| **APOG1** | Hs.110675 | **CSTA** | Hs.518198 |
| **BIK** | Hs.475055 | **CX3CL1** | Hs.531668 |
| **HOXC6** | Hs.820 | **GJA1** | Hs.74471 |
| **CDK5** | Hs.166071 | **BNIP2** | Hs.283454 |
| **AMACR** | Hs.508343 | **PER2** | Hs.58756 |
| **LASS2** | Hs.285976 | **EPHA2** | Hs.171596 |
| **HPN** | Hs.182385 | **FOX01A** | Hs.370666 |
| **NME1** | Hs.118638 | **FOXF1** | Hs.155591 |
| **PRDX4** | Hs.83383 | | |
| **GJB1** | Hs.333303 | | |
| **SYNGR2** | Hs.464210 | | |
| **SIM2** | Hs.146186 | | |
| **EIF3S2** | Hs.530096 | | |
| **NIT2** | Hs.439152 | | |
| **FOXA1** | Hs.163484 | | |
| | | | |

For these determinations, the microdissected material consisted exclusively of pure epithelial cells, taken either from tumors or from normal prostate tissue.

This first carefully selected subset of 45 genes provided a high capacity to discriminate between normal and carcinomatous samples. The selection of these genes was based on three criteria: (1) the capacity of each gene to discriminate between normal and carcinomatous samples in the expression analysis on Affymetrix HGF microarrays (values from Table 6), i.e. genes having the most significant p values; (2) the biological interest thereof, based on functional and expression data previously described in the scientific literature; and (3), as far as possible, the existence of commercial antibodies specific for the corresponding proteins, for subsequent validation of expression by means of immunoassays, including immunohistochemical determinations.

In fact, this subset of genes correctly includes within the group of carcinomatous samples a sample that had been incorrectly grouped together with global transcriptomic analysis by means of FADA (Figure 2).

More specifically, in the case of microdissected samples it was found by this method that, of the 26 genes included in Diagnostic Chip 1 that were considered to be overexpressed in tumors according to Affymetrix HGF microarray analysis, 13 genes (50%) also exhibited higher levels in tumors than in noncarcinomatous tissue in quantitative determination by real-time RT-PCR. In the case of the 19 genes found underexpressed in tumors by microarray determination, of the 18 genes that were detectable, 18 (95%) were found underexpressed by real-time RT-PCR in the analysis of non-microdissected samples. When the quantitative determination was performed on microdissected samples (i.e. comparing carcinomatous pure epithelia with normal pure epithelia from the same individuals), it emerged that, of the 26 genes selected as overexpressed in tumors, only 9 (34.6%) were also found overexpressed in the majority of samples by means of transcript quantification by real-time RT-PCR. In this determination on microdissected samples, of the 19 genes considered as underexpressed in tumors following the microarray analyses, 18 were assessable and, of these, 15 (83.3%) were also found underexpressed in most microdissected samples using quantitative determination by real-time RT-PCR. Therefore, of the 45 assessable genes on Diagnostic Chip 1 (26 overexpressed and 19 underexpressed), 24 (9 overexpressed and 15 underexpressed) had their respective expression profiles validated by real-time RT-PCR on laser-microdissected pure epithelia. Taking into account the results obtained in the validations with non-microdissected samples and with microdissected samples, genes that had been validated in both analyses were selected, resulting in a set of 22 genes (7 overexpressed and 15 underexpressed; see Table 4). Taking the expression data from the Affymetrix HGF microarray analysis corresponding to these 22 genes, it was found that this small subset of expression data allows perfect differentiation between carcinomatous and normal samples with high statistical significance (Figure 4).

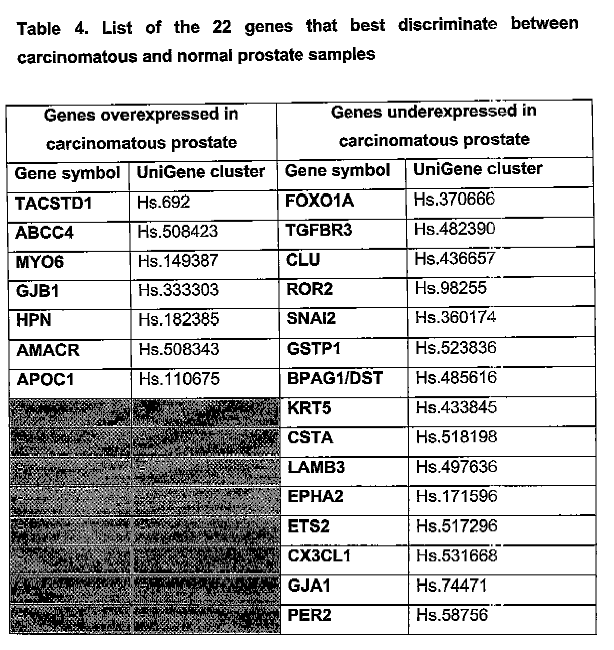

Using even stricter real-time RT-PCR validation criteria for selecting genes overexpressed or underexpressed in tumors, and taking into account the compartments in which it had been deduced from their expression profiles that each gene was expressed, it was possible to identify an even smaller subset of 14 genes (6 overexpressed in tumors and 8 underexpressed; see Table 5). Again taking the expression data corresponding to these 14 genes obtained for all the starting samples on Affymetrix HGF microarrays, it was found that this smaller subset was also able to discriminate with high statistical significance between carcinomatous samples and normal prostate samples (Figure 5).

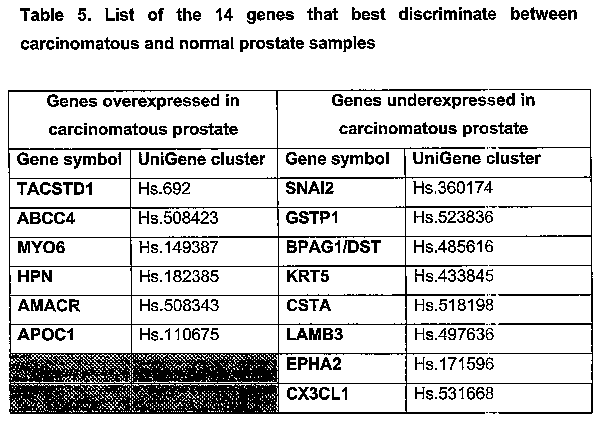

One of the applications for the gene sets whose expression profiles are capable of discriminating between carcinomatous samples and their normal counterparts is that of predicting whether a prostate tissue sample is carcinomatous or not, a diagnosis that could not have been known in advance. A prerequisite for being able to apply this type of predictive analysis is that said gene set must be capable of discriminating between carcinomatous and noncarcinomatous samples, not only on the basis of the experimental data themselves, but also on the basis of the experimental data of others. In order to discover what was the minimum set of genes, from among the set of 14 genes described above, having sufficient capacity to discriminate between carcinomatous and noncarcinomatous samples, a linear discriminant analysis (LDA) was performed [64]. This is a statistical technique that allows objects to be exhaustively classified into mutually exclusive groups, based on sets of measurable characteristics of such objects. In this case, the point was to classify samples into carcinomatous and noncarcinomatous, using the expression levels of given sets of genes as measurable variables. The ultimate objective was to optimize the set of genes most useful for discriminating between carcinomatous and normal samples. In order to extend the usefulness of this classifying set beyond the 27 experimental samples, data corresponding to another microarray analysis carried out on 57 samples, published by Liu *et al.* [65], were obtained. In order to be able to apply statistical analysis equally to all the samples, expression data from the 84 samples (27 own samples and the 57 of Liu *et al*.) were normalized using the RMA method of Irizarry *et al.* [14], followed by quantile normalization. Next, the samples were randomly distributed into two groups: a training group of 63 samples (75% of all the samples) and a validation, or test, group of 21 samples. Using the training group, all the possible gene-pair combinations from among the 14 genes described above were applied in a cross-validation of the LOOCV type ("leave-one-out cross-validation"), which quantitates the capacity to discriminate between carcinomatous and normal samples when applying LDA as implemented in the R MASS package [66]. From this LOOCV analysis it was found that the gene pair comprising TACSTD1 and LAMB3 was capable of classifying samples correctly as carcinomatous or normal in 98% of cases. Accordingly, this gene pair was used as the starting point for increasing, in increments of one, the number of genes (from among the 14-gene set or mini-signature), keeping those that gave the best results in the LOOCV test. This process led to a minimum set of seven genes from the mini-signature of 14, which allowed carcinomatous and normal samples to be classified with complete accuracy in an LOOCV analysis, and this worked equally well with data relating to our own samples and to the data of Liu *et a*/*.* These genes are, from among those overexpressed in tumors: TACSTD1, MYO6, and HPN, and from among those underexpressed in tumors: LAMB3, EPHA2, DST, and CSTA.

**Table 6. LDA weights for each of the seven genes in the minimum classifying set**

| **Gene** | **TACSTD 1** | **MYO6** | **EPHA2** | **DST** | **HPN** | **CSTA** | **LAMB3** |
|---|---|---|---|---|---|---|---|
| Weight | 1.0737 | -0.1341 | 0.5108 | -0.0248 | 0.7580 | 0.2182 | -1.9292 |

### Cut-off point: 7.93

Similarly, a series of 27 paired human prostate samples - i.e. carcinomatous samples and the corresponding normal samples from the same patient - were analyzed by hybridization on 60-mer oligonucleotide microarrays in which the entire human transcriptome was represented. The grading of the carcinomatous samples according to the Gleason scoring system was as follows: 5 samples in Grade 5, 2 samples in Grade 6, 15 samples in Grade 7, 2 samples in Grade 8, and 2 samples in Grade 9. At the same time, 3 samples of stromal tissue were also analyzed. The paired samples were cohybridized after labeling with different fluorochromes. The stroma samples were cohybridized against a pool of normal samples.

This analysis made it possible to identify a set of 15 genes, in addition to the 45 genes identified previously, that would also make it possible to discriminate between carcinomatous samples and normal samples. In particular, this set was made up of the genes CRYAB, CYP27A1, FGF2, IKL, PTGIS, RARRES2, PLP2, TPM2, S100A6, SCHIP1, GOLPH2, TRIM36, POLD2, CGREF1, and HSD17B4. Of these, the genes GOLPH2, TRIM36, POLD2, CGREF1, and HSD17B4 were overexpressed, while the genes CRYAB, CYP27A1, FGF2, IKL, PTGIS, RARRES2, PLP2, TPM2, S100A6, and SCHIP1 were underexpressed.

In this way, a set of 60 genes was defined that exhibited a high capacity to discriminate between carcinomatous samples and normal samples.

### Example 3 - Immunohistochemical technique used on tissue microarrays.

The tissue microarrays were constructed using a Beecher instrument (Beecher Instruments) and a 1 mm-diameter needle. Three different microarrays were constructed, containing selected zones of samples of normal prostate, carcinomatous prostate, and PIN tissue, all previously embedded in paraffin. Blocks of lung tissue previously stained with three different colors and placed in different zones of the microarray were used as orientation markers for the samples within the arrays. Complete sections of the microarrays were taken and stained with hematoxylin-eosin to confirm quality. 2 µm thick sections were taken and mounted on xylene-coated glass slides (Dako, Carpinteria, CA) for the immunohistochemical stainings. These were done with the Techmate 500 system (Dako), using the Envision system (Dako) for the detection. Briefly, the sections were deparaffinized and rehydrated in graded alcohol series and water. For the detection of MY06, antigen unmasking was performed in a pressure cooker with citrate buffer (pH 6) for 5 min. This treatment was not done for the EPHA2 and CX3CL1 antigens. Next, the microarrays were incubated for 30 min with the primary antibodies (1:100 dilution for MYO6, mouse monoclonal antibody from Sigma, St. Louis, MO; 1:50 dilution for EPHA2, mouse monoclonal antibody from Sigma; and 1:200 dilution for CX3CL1, goat polyclonal antibody from R&D Systems, Minneapolis, MN) and washed in ChemMate buffer solution (Dako). The endogenous peroxidase was blocked for 7.5 min in ChemMate peroxidase-blocking solution and then incubated for 30 min with a peroxidase-labeled polymer. After washing in ChemMate buffer solution, the microarrays were incubated with the chromogenic substrate solution diaminobenzidine, washed in water, counterstained with hematoxylin, dehydrated, and mounted.

The results were analyzed by a pathologist. Two aspects of the immunohistochemistries were analyzed: firstly, the percentage of epithelial staining, assessed as between 0 and 100%, and secondly, the intensity of the staining, assessed as none (0), weak (1), moderate (2), or intense (3). The expression patterns of each of the proteins were also analyzed. A sample was considered to exhibit overexpression or underexpression of a protein by comparison with another sample when the percentage difference in epithelial staining between the two samples was greater than 20% and/or the intensity was different by at least one grade.

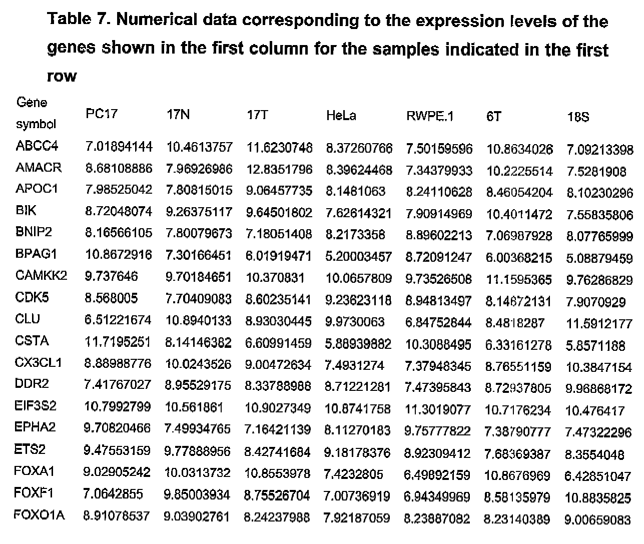

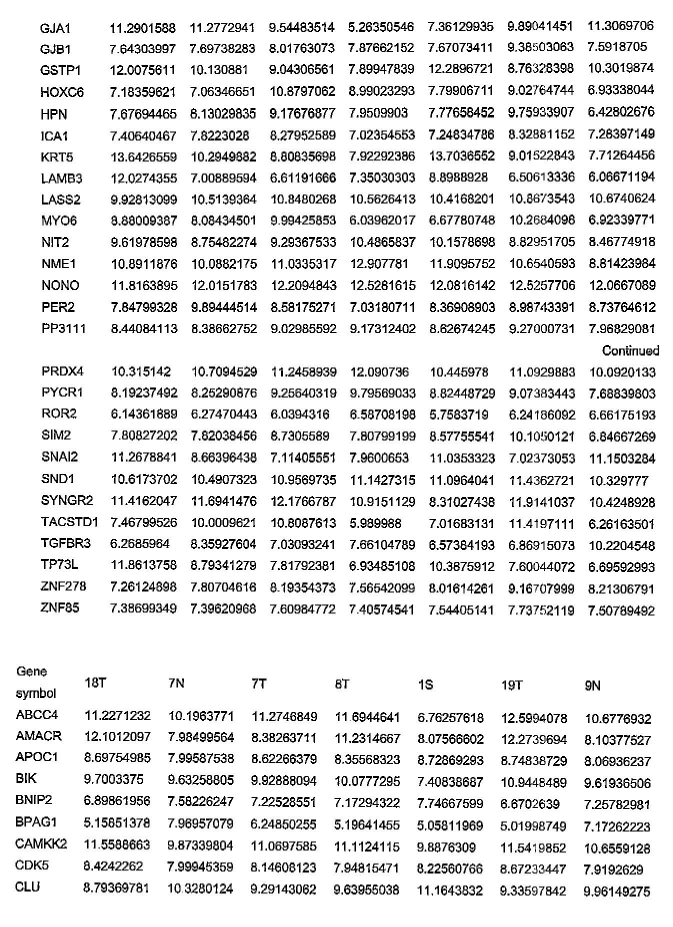

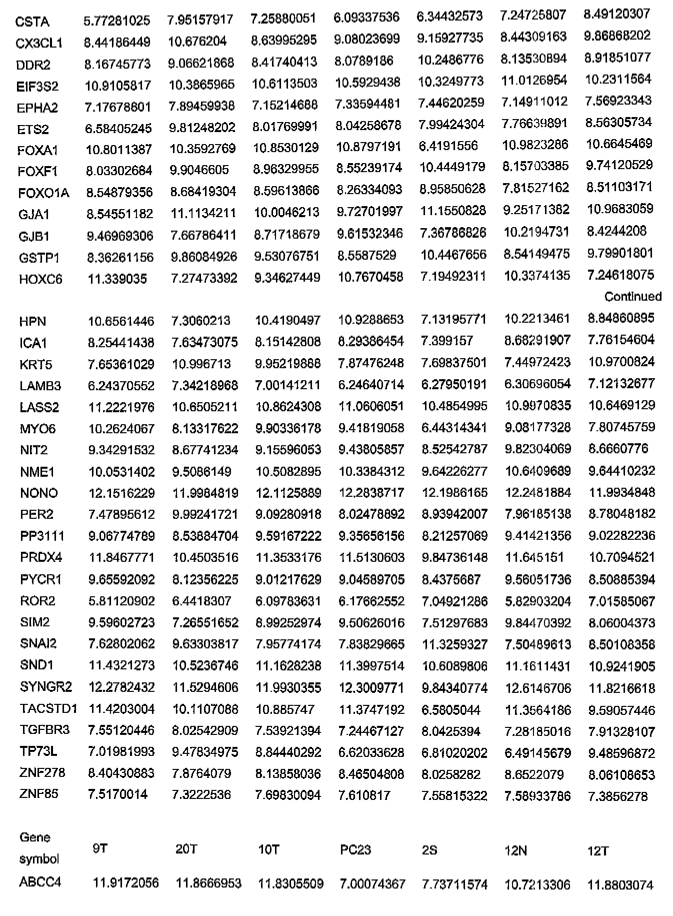

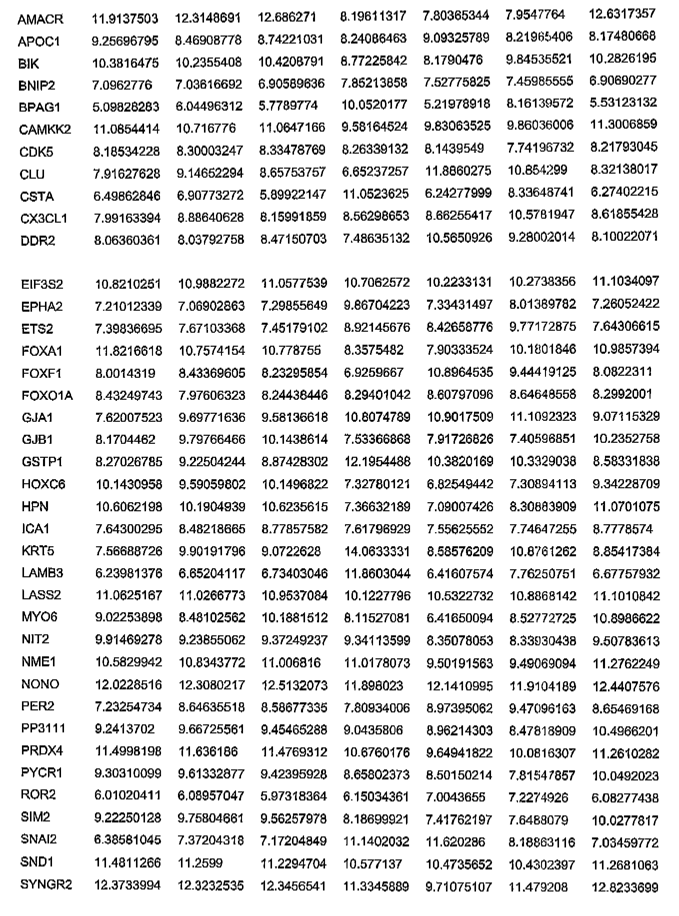

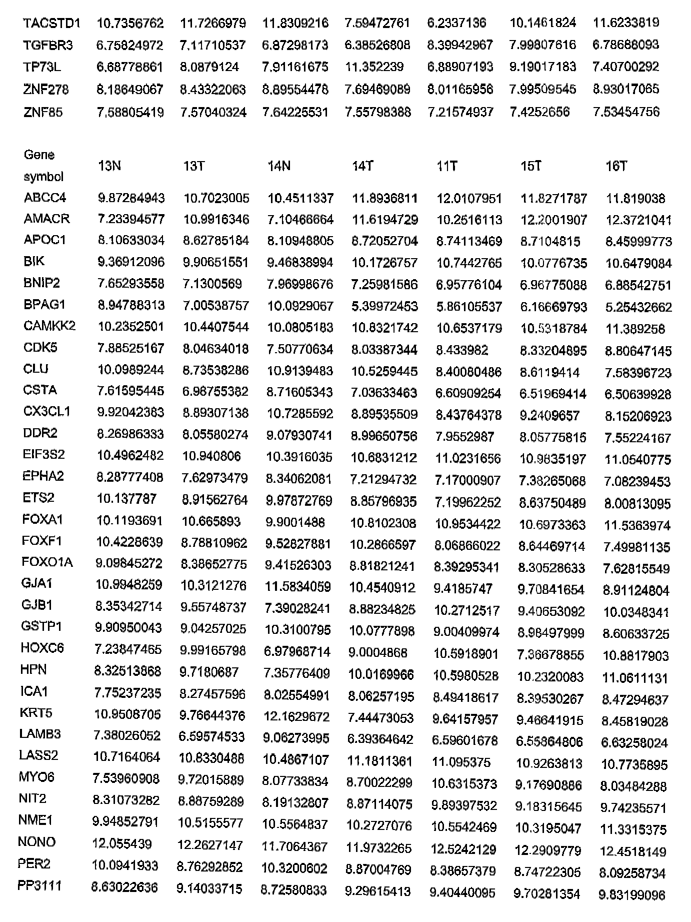

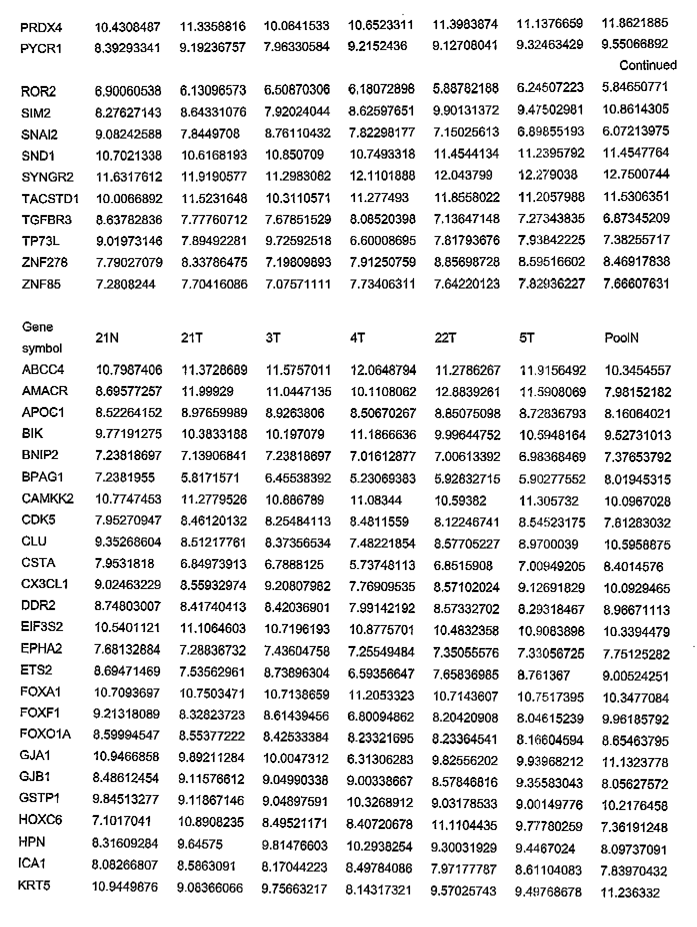

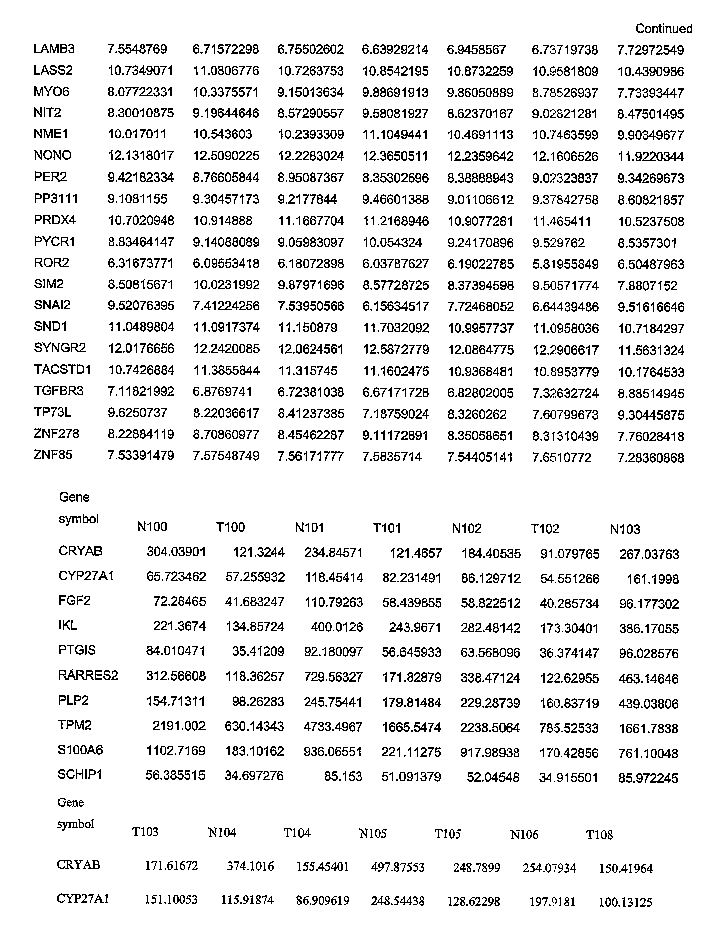

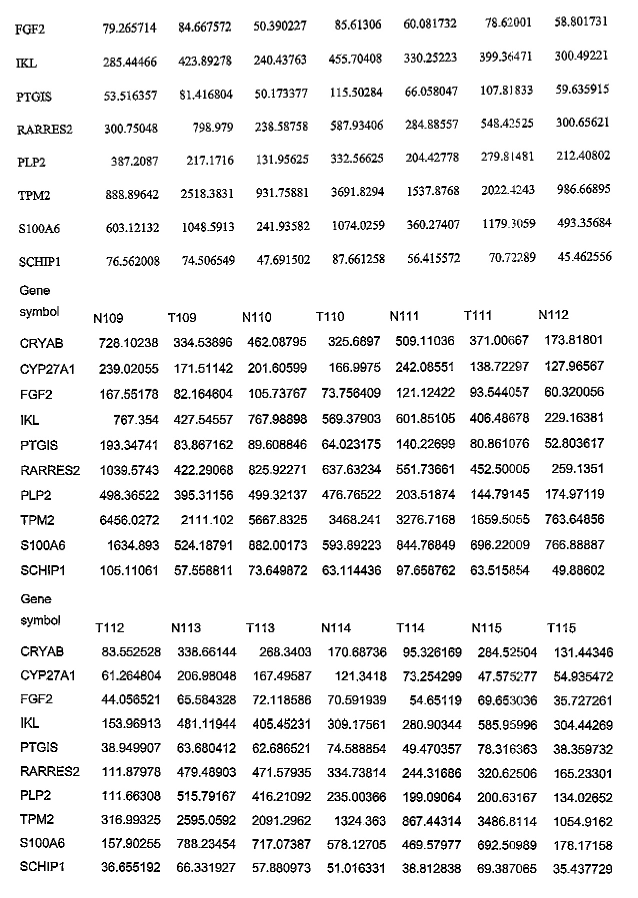

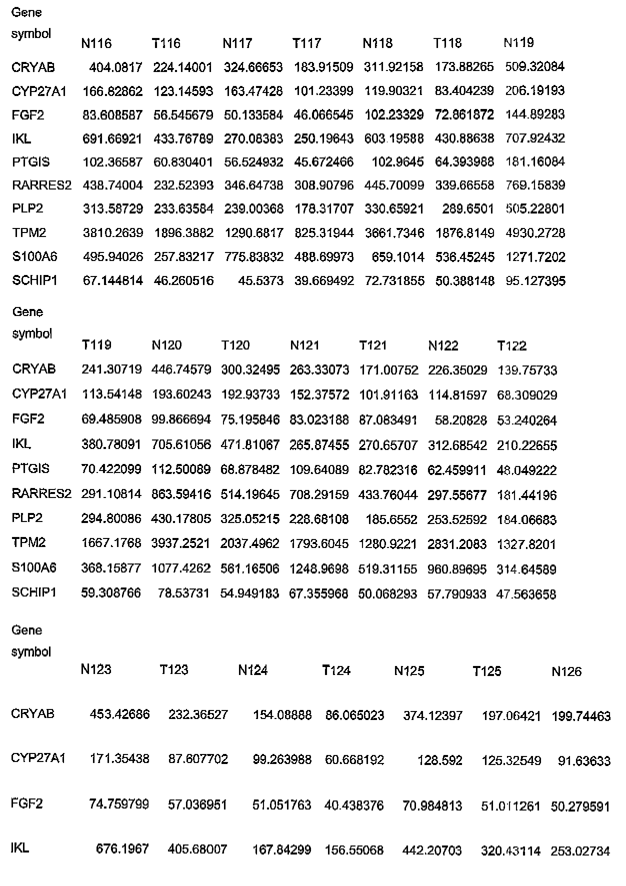

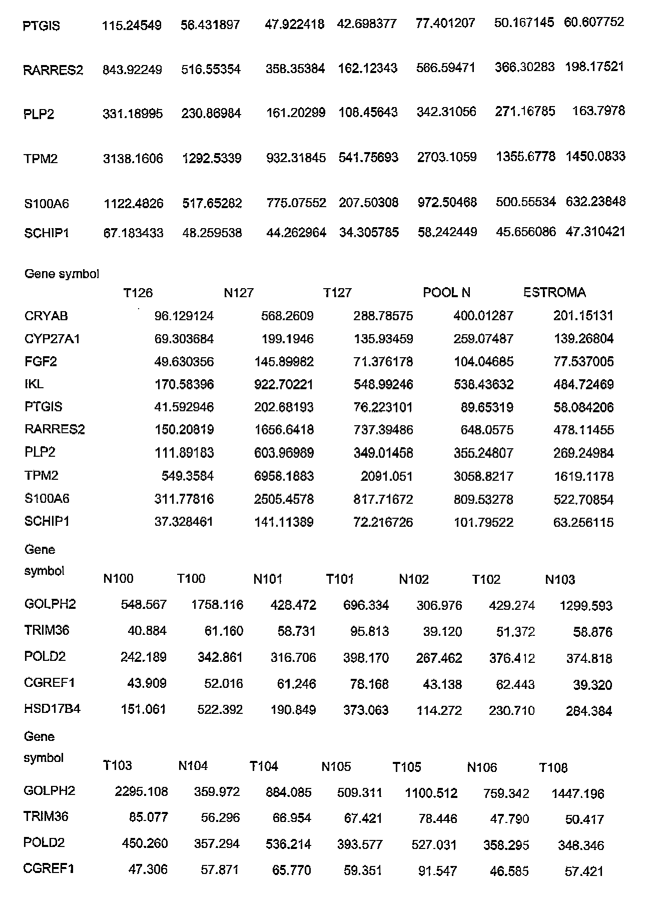

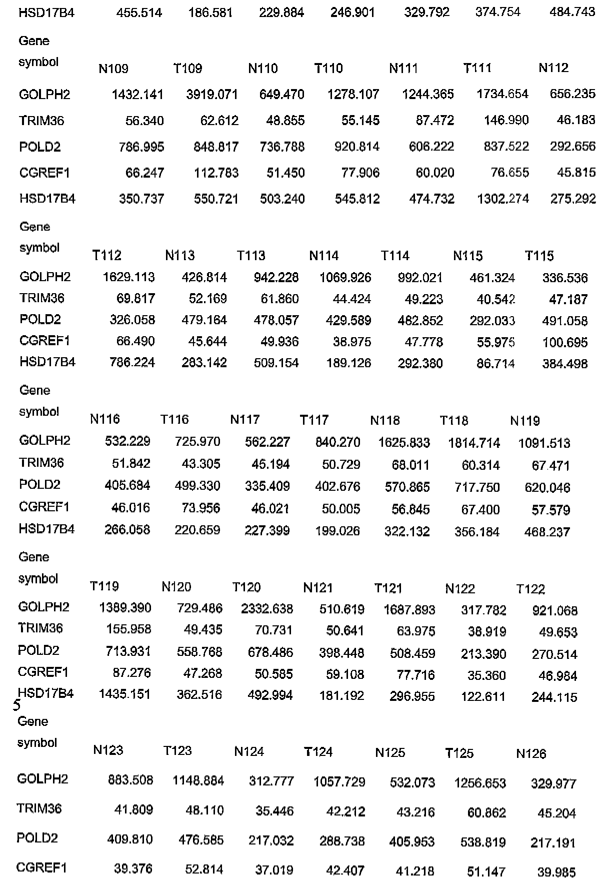

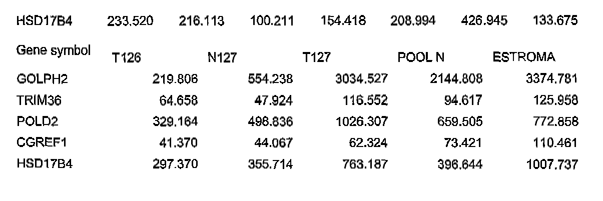

### REFERENCES

**1.** Brawley OW, et al. (1998). The epidemiology of prostate cancer part I: descriptive epidemiology. Semin. Urol. Oncol. 16, 187-192.
**2.** Simard J, et al. (2002). Perspective: Prostate cancer susceptibility genes. Endocrinology 143, 2029-2040.
**3.** DeMarzo AM, Nelson WG, Isaacs WB, Epstein JI (2003). Pathological and molecular aspects of prostate cancer. Lancet 361, 955-964.
**4.** Rubin MA, et al. (2004). Quantitative determination of expression of the prostate cancer protein □-methylacyl-CoA racemase using automated quantitative analysis (AQUA). Am. J. Pathol. 163, 831-840.
**5.** van't Veer LJ, et al. (2002). Gene expression profiling predicts clinical outcome of breast cancer. Nature 415, 530-536.
**6.** van de Vijver MJ, et al. (2002). A gene-expression signature as a predictor of survival in breast cancer. N. Engl. J. Med. 347, 1999-2009.
**7.** Lapointe J, et a/. (2004). Gene expression profiling identifies clinically relevant subtypes of prostate cancer. Proc. Natl. Acad. Sci. USA 101, 811-816.
**8.** Rhodes DR, et a/. (2002). Meta-analysis of microarrays: interstudy validation of gene expression profiles reveals pathway dysregulation in prostate cancer. Cancer Res. 62, 4427-4433.
**9.** Sancho E, et al. (1998). Role of UEV-1, an inactive variant of the E2 ubiquitin-conjugating enzymes, in in vitro differentiation and cell cycle behavior of HT-29-M6 intestinal mucosecretory cells. Mol. Cell. Biol, 18, 576-589.
**10.** Benedit P, et al. (2001). PTOV1, a novel protein overexpressed in prostate cancer containing a new class of protein homology blocks. Oncogene 20, 1455-1464.
**11.** Santamaria A, et al. (2003). PTOV-1, a novel protein overexpressed in prostate cancer, shuttles between the cytoplasm and the nucleus and promotes entry into the S phase of the cell division cycle. Am. J. Pathol. 162, 897-905.
**12.** Santamaria A, et al. (2005). PTOV1 enables the nuclear translocation and mitogenic activity of Flotillin-1, a major protein of lipid rafts. Mol. Cell. Biol. 25, 1900-1911.
**13.** Lozano JJ, et al. (2005). Dual activation of pathways regulated by steroid receptors and peptide growth factors in primary prostate cancer revealed by Factor Analysis of microarray data. BMC Genomics 6, 109.
**14.** Irizarry, R. A., et al. (2003). Exploration, normalization, and summaries of high density oligonucleotide array probe level data. Biostatistics, 4, 249-264.
**15.** Bolstad, B. M., et al. (2003). A comparison of normalization methods for high density oligonucleotide array data based on variance and bias. Bioinformatics 19, 185-193.
**16.** Tukey, J. W. (1977). Exploratory data analysis. Addison-Wesley.
**17.** Storey, J. D. and Tibshirani, R. (2003). Statistical significance for genomewide studies. Proc Natl Acad Sci USA 100, 9440-9445.
**18.** Benjamini, Y., y Hochberg, Y. (1995). Controlling the False Discovery Rate - a practical and powerful approach to multiple testing. Journal of the Royal Statistical Society Series B-Methodological, 57, 289-300.
**19.** Magee JA, et al.(2001).Expression profiling reveals hepsin overexpression in prostate cancer. Cancer Res.61, 5692-5696.
**20.** Welsh JB, et al. (2001). Analysis of gene expression identifies candidate markers and pharmacological targets in prostate cancer. Cancer Res. 61, 5974-5978.
**21.** Dhanasekaran SM, et al. (2001), Delineation of prognostic biomarkers in prostate cancer. Nature 412, 822-826.
**22.** Klezovitch O, et al. (2004).Hepsin promotes prostate cancer progression and metastasis. Cancer Cell 6, 185-195.
**23.** Jiang Z, et al. (2001). P504S: a new molecular marker for the detection of prostate carcinoma. Am. J. Surg. Pathol. 25, 1397-1404.
**24.** Rubin MA, et al. (2002). alpha-Methylacyl coenzyme A racemase as a tissue biomarker for prostate cancer. JAMA 287, 1662-1670.
**25.** Luo J, et al. (2002). Alpha-methylacyl-CoA racemase: a new molecular marker for prostate cancer. Cancer Res. 62, 2220-2226.
**26.** Beach R, et al. (2002). P504S immunohistochemical detection in 405 prostatic specimens including 376 18-gauge needle biopsies. Am. J. Surg. Pathol. 26, 1588-1596.
**27.** Evans AJ (2003). Alpha-methylacyl CoA racemase (P504S): overview and potential uses in diagnostic pathology as applied to prostate needle biopsies. J. Clin. Pathol. 56, 892-897.
**28.** Jiang Z, Woda BA (2004). Diagnostic utility of alpha-methylacyl CoA racemase (P504S) on prostate needle biopsy. Adv. Anat. Pathol. 11, 316-321.
**29.** DeYoung MP, et al.(2003). Identification of Down's syndrome critical locus gene SIM2-s as a drug therapy target for solid tumors. Proc. Natl. Acad. Sci. USA 100, 4760-4765.
**30.** Ratan RR (2003). Mining genome databases for therapeutic gold: SIM2 is a novel target for treatment of solid tumors. Trends Pharmacol. Sci. 24, 508-510.
**31.** Aleman MJ, et al.(2005). Inhibition of Single Minded 2 gene expression mediates tumor-selective apoptosis and differentiation in human colon cancer cells. Proc. NatI. Acad. Sci. USA 102, 12765-12670.
**32.** Bijl J, et a/. (1996). Expression of HOXC4, HOXC5, and HOXC6 in human lymphoid cell lines, leukemias, and benign and malignant lymphoid tissue. Blood 87, 1737-1745.
**33.** Alami Y, et al. (1999). HOXC5 and HOXC8 expression are selectively turned on in human cervical cancer cells compared to normal keratinocytes. Biochem. Biophys. Res. Commun. 257, 738-745.
**34.** Waltregny D, et al. (2002). Overexpression of the homeobox gene HOXC8 in human prostate cancer correlates with loss of tumor differentiation. Prostate 50, 162-169.
**35.** Miller GJ, et al. (2003). Aberrant HOXC expression accompanies the malignant phenotype in human prostate. Cancer Res. 63, 5879-5888.
**36.** Ramachandran S, et al. (2005). Loss of HOXC6 expression induces apoptosis in prostate cancer cells. Oncogene 24, 188-198.
**37.** Lee WH, et al. (1994). Cytidine methylation of regulatory sequences near the pi-class glutathione S-transferase gene accompanies human prostatic carcinogenesis. Proc. Natl. Acad. Sci. USA 91, 11733-11737.
**38.** Lin X, et al. (2001). GSTP1 CpG island hypermethylation is responsible for the absence of GSTP1 expression in human prostate cancer cells. Am. J. Pathol, 159, 1815-1826.
**39.** Yang A, et al.(2002). On the shoulders of giants: p63, p73 and the rise of p53. Trends Genet. 18, 90-95.
**40.** Signoretti S, et al. (2000). p63 is a prostate basal cell marker and is required for prostate development. Am. J. Pathol. 157, 1769-1775.
**41.** Yoshida H, et al. (2004). Lessons from border cell migration in the Drosophila ovary: A role for myosin VI in dissemination of human ovarian cancer. Proc. NatI. Acad. Sci. USA 101, 8144-8149.
**42.** Varambally S, et a/. (2005). Integrative genomic and proteomic analysis of prostate cancer reveals signatures of metastatic progression. Cancer Cell 8, 393-406.
**43.** Andres AC, et al. (1994). Expression of two novel eph-related receptor protein tyrosine kinases in mammary gland development and carcinogenesis. Oncogene 9, 1461-1467.
**44.** Nemoto T, et a/. (1997). Overexpression of protein tyrosine kinases in human esophageal cancer. Pathobiology 65, 195-203.
45. Walker-Daniels J, et al. (1999)Overexpression of the EphA2 tyrosine kinase in prostate cancer. Prostate 41, 275-280.
**46.** Ogawa K, et al. (2000). The ephrin-A1 ligand and its receptor, EphA2, are expressed during tumor neovascularization. Oncogene 19, 6043-6052.
**47.** Zelinski DP, et al. (2001). EphA2 overexpression causes tumorigenesis of mammary epithelial cells. Cancer Res. 61, 2301-2306.
**48.** Straume O, Akslen LA (2002). Importance of vascular phenotype by basic fibroblast growth factor, and influence of the angiogenic factors basic fibroblast growth factor/fibroblast growth factor receptor-1 and ephrin-A1/EphA2 on melanoma progression. Am. J. Pathol. 160, 1009-1019.
**49.** Miyazaki T, et a/. (2003). EphA2 overexpression correlates with poor prognosis in esophageal squamous cell carcinoma.Int. J. Cancer 103,657-663.
**50.** Kinch MS, et a/. (2003). Predictive value of the EphA2 receptor tyrosine kinase in lung cancer recurrence and survival. Clin. Cancer Res. 9, 613-618.
**51.** Zeng G, et al. (2003). High-level expression of EphA2 receptor tyrosine kinase in prostatic intraepithelial neoplasia. Am. J. Pathol. 163, 2271-2276.
**52.** Koffman KT, et a/. (2003). Differential EphA2 epitope display on normal versus malignant cells. Cancer Res. 63, 7907-7912.
**53.** Saito T, et a/. (2004). Expression of EphA2 and E-cadherin in colorectal cancer: correlation with cancer metastasis. Oncol. Rep. 11, 605-611.
**54.** Duxbury MS, et a/. (2004). EphA2: a determinant of malignant cellular behavior and a potential therapeutic target in pancreatic adenocarcinoma. Oncogene 23, 1448-1456.
**55.** Fox BP, Kandpal RP (2004). Invasiveness of breast carcinoma cells and transcript profile: Eph receptors and ephrin ligands as molecular markers of potential diagnostic and prognostic application. Biochem. Biophys. Res. Commun. 318, 882-892.
**56.** Wu D, et al. (2004). Prognostic value of EphA2 and EphrinA-1 in squamous cell cervical carcinoma.Gynecol. Oncol. 94, 312-319.
**57.** Thaker PH, et al. (2004). EphA2 expression is associated with aggressive features in ovarian carcinoma. Clin. Cancer Res. 10, 5145-5150.
**58.** Nakamura R, et al. (2005). EPHA2/EFNA1 expression in human gastric cancer. Cancer Sci. 96, 42-47.
**59.** Herrem CJ, et al. (2005). Expression of EphA2 is prognostic of disease-free interval and overall survival in surgically treated patients with renal cell carcinoma. Clin. Cancer Res. 11, 226-231.
**60.** Hajra KM, et al. (2002). The SLUG zinc-finger protein represses E-cadherin in breast cancer. Cancer Res. 62, 613-1618.
**61.** Uchikado Y, et al. (2005). Slug expression in the E-cadherin preserved tumors is related to prognosis in patients with esophageal squamous cell carcinoma. Clin. Cancer Res. 11, 1174-1180.
**62.** Elloul S, et al. (2005). Snail, Slug, and Smad-interacting protein 1 as novel parameters of disease aggressiveness in metastatic ovarian and breast carcinoma. Cancer 103, 1631-1643.
**63.** Martin TA, et al. (2005).Expression of the transcription factors snail, slug, and twist and their clinical significance in human breast cancer. Ann. Surg. Oncol. 12, 488-496.
**64.** Venables, W. N. & Ripley, B. D. (2002). Modern Applied Statistics with S. Fourth Edition. Springer, New York.
**65.** Liu, P., et a/. (2006). Sex-Determining Region Y Box 4 is a Transforming Oncogene in Human Prostate Cancer Cells. Cancer Research 66, 4011-4019.
**66.** R Development Core Team (2006). R: A language and environment for statistical computing. R Foundation for Statistical Computing, Vienna, Austria. http://www.R-project.org.

## Claims

1. A method for the molecular diagnosis of prostate cancer comprising the *in vitro* analysis, in a test sample, of the expression level of at least two genes selected from the group consisting of: TACSTD1, HPN, AMACR, APOC1, GJB1, PP3111, CAMKK2, ZNF85, SND1, NONO, ICA1, PYCR1, ZNF278, BIK, HOXC6, CDK5, LASS2, NME1, PRDX4, SYNGR2, SIM2, EIF3S2, NIT2, FOXA1, CX3CL1, SNAI2, GSTP1, DST, KRT5, CSTA, LAMB3, EPHA2, GJA1, PER2, FOXO1A, TGFBR3, CLU, ROR2, ETS2, TP73L, DDR2, BNIP2, FOXF1, MYO6, ABCC4, CRYAB, CYP27A1, FGF2, IKL, PTGIS, RARRES2, PLP2, TPM2, S100A6, SCHIP1, GOLPH2, TRIM36, POLD2, CGREF1, and HSD17B4, wherein the capacity to discriminate between carcinomatous and noncarcinomatous samples when the expression levels of two or more genes from said group are determined together is greater than the discriminating capacity of the same genes separately.

2. The method as claimed in claim 1, wherein the genes are selected from the group consisting of: TACSTD1, HPN, AMACR, APOC1, GJB1, CX3CL1, SNAI2, GSTP1, DST, KRT5, CSTA, LAMB3, EPHA2, GJA1, PER2, FOXO1A, TGFBR3, CLU, ROR2, ETS2, MYO6, and ABCC4, and wherein the capacity to discriminate between carcinomatous and noncarcinomatous samples when the expression levels of two or more genes from said group are determined together is greater than the discriminating capacity of the same genes separately.

3. The method as claimed in either of claims 1 to 2, wherein the genes are selected from the group consisting of: TACSTD1, HPN, AMACR, APOC1, CX3CL1, SNAI2, GSTP1, DST, KRT5, CSTA, LAMB3, EPHA2, MYO6 and ABCC4, and wherein the capacity to discriminate between carcinomatous and noncarcinomatous samples when the expression levels of two or more genes from said group are determined together is greater than the discriminating capacity of the same genes separately.

4. The method as claimed in any one of claims 1 to 3, wherein the genes are selected from the group consisting of: TACSTD1, HPN, DST, CSTA, LAMB3, EPHA2, and MYO6 and wherein the capacity to discriminate between carcinomatous and noncarcinomatous samples when the expression levels of two or more genes from said group are determined together is greater than the discriminating capacity of the same genes separately.

5. The method as claimed in any one of claims 1 to 4, wherein at least one of the genes selected from the group is the gene MY06.

6. The method as claimed in any one of claims 1 to 3, wherein at least one of the genes selected from the group is the gene ABCC4.

7. The method as claimed in claim 5, wherein the analysis of the expression level of the gene MYO6 is combined with the analysis of the expression level of at least one gene from the group consisting of: ABCC4, AMACR, BIK, BNIP2, CDK5, CSTA, DST, EIF3S2, EPHA2, ETS2, GJB1, HPN, NIT2, PYCR1, ROR2, TACSTD1, and TP73L.

8. The method as claimed in claim 6, wherein the analysis of the expression level of the gene ABCC4 is combined with the analysis of the expression level of at least one gene from the group consisting of: CSTA, GJB1, GSTP1, HOXC6, HPN, LAMB3, MYO6, PRDX4, and TP73L.

9. The method as claimed in any one of claims 1 to 8, wherein the analysis of the expression level of said genes is performed by determining the level of mRNA derived from their transcription.

10. The method as claimed in claim 9, wherein the analysis is performed by means of amplification by PCR, RT-PCR, RT-LCR, SDA, or any other method of nucleic acid amplification.

11. The method as claimed in claim 9, wherein the analysis is performed by DNA chips produced with oligonucleotides deposited by any procedure.

12. The method as claimed in claim 9, wherein the analysis is performed by DNA chips produced with oligonucleotides synthesized *in situ* by means of photolithography or by any other procedure.

13. The method as claimed in claim 9, wherein the analysis is performed by *in situ* hybridization using specific probes labeled by any labeling method.

14. The method as claimed in claim 9, wherein the analysis is performed by gel electrophoresis.

15. The method as claimed in claim 14, wherein the analysis is performed by means of membrane transfer and hybridization with a specific probe.

16. The method as claimed in claim 9, wherein the analysis is performed by means of NMR or any other diagnostic imaging technique.

17. The method as claimed in claim 9, wherein the analysis is performed by means of NMR or any other diagnostic imaging technique using paramagnetic nanoparticles or any other type of detectable nanoparticles functionalized with antibodies or by any other means.

18. The method as claimed in any one of claims 1 to 8, wherein the analysis of the expression level of said genes is performed by determining the level of protein encoded by the gene or fragments thereof.

19. The method as claimed in claim 18, wherein the analysis is performed by means of incubation with a specific antibody.

20. The method as claimed in claim 19, wherein the analysis is performed by means of the Western blot method.

21. The method as claimed in claim 19, wherein the analysis is performed by means of immunohistochemistry.

22. The method as claimed in claim 18, wherein the analysis is performed by means of gel electrophoresis.

23. The method as claimed in claim 18, wherein the analysis is performed by means of protein chips.

24. The method as claimed in claim 18, wherein the analysis is performed by means of ELISA or any other enzymatic method.

25. The method as claimed in claim 18, wherein the analysis is performed by means of NMR or any other diagnostic imaging technique.

26. The method as claimed in claim 18, wherein the analysis is performed by means of NMR or any other diagnostic imaging technique using paramagnetic nanoparticles or any other type of detectable nanoparticles functionalized with antibodies or by any other means.

27. A kit for the molecular diagnosis of prostate cancer, comprising the appropriate reagents and additives for detecting variations in the expression levels of genes in accordance with the method according to any one of claims 1 to 26.

28. The method as claimed in any one of claims 1 to 4, wherein the overexpression of the genes MYO6 TACSTD1, or HPN, or the underexpression of the genes DST, CSTA, LAMB3, or EPHA2 is used for the diagnosis of the presence of prostate cancer or of a premalignant condition thereof, or for the prognosis of the progression of the prostate cancer or of a premalignant condition thereof, or for the prognosis of the risk of recurrence of said disease.

29. The method as claimed in any one of claims 1 to 3, wherein the overexpression of the genes MYO6, ABCC4, TACSTD1, HPN, AMACR, or APOC1, or the underexpression of the genes CX3CL1, SNAI2, GSTP1, DST, KRT5, CSTA, LAMB3, or EPHA2 is used for the diagnosis of the presence of prostate cancer or of a premalignant condition thereof, or for the prognosis of the progression of the prostate cancer or of a premalignant condition thereof, or for the prognosis of the risk of recurrence of said disease.

30. The method as claimed in any one of claims 1 to 2, wherein the overexpression of the genes MYO6, ABCC4, TACSTD1, HPN, AMACR, APOC1, or GJB1, or the underexpression of the genes CX3CL1, SNAI2, GSTP1, DST, KRT5, CSTA, LAMB3, EPHA2 , GJA1, PER2, FOXO1A, TGFBR3, CLU, ROR2, or ETS2 is used for the diagnosis of the presence of prostate cancer or of a premalignant condition thereof, or for the prognosis of the progression of the prostate cancer or of a premalignant condition thereof, or for the prognosis of the risk of recurrence of said disease.

31. The method as claimed in claim 1, wherein the overexpression of the genes MYO6, ABCC4, TACSTD1, HPN, AMACR, APOC1, GJB1, PP3111, CAMKK2, ZNF85, SND1, NONO, ICA1, PYCR1, ZNF278, BIK, HOXC6, CDK5, LASS2, NME1, PRDX4, SYNGR2, SIM2, EIF3S2, NIT2, FOXA1, GOLPH2, TRIM36, POLD2, CGREF1, or HSD17B4, or the underexpression of the genes CX3CL1, SNAI2, GSTP1, DST, KRT5, CSTA, LAMB3, EPHA2, GJA1, PER2, FOXO1A, TGFBR3, CLU, ROR2, ETS2, TP73L, DDR2, BNIP2, FOXF1, CRYAB, CYP27A1, FGF2, IKL, PTGIS, RARRES2, PLP2, TPM2, S100A6, or SCHIP1 is used for the diagnosis of the presence of prostate cancer or of a premalignant condition thereof, or for the prognosis of the progression of the prostate cancer or of a premalignant condition thereof, or for the prognosis of the risk of recurrence of said disease.

32. The method as claimed in any one of claims 1 to 4, wherein the discriminating capacity when the expression levels of two or more genes are determined together is 1%, preferably 10%, more preferably 25%, more preferably still 50% greater than the discriminating capacity of at least one of the genes separately.
